(19) Europäisches Patentamt
European Patent Office
Office européen des brevets

(11) EP 1 534 687 B1

# (12) EUROPEAN PATENT SPECIFICATION

(45) Date of publication and mention of the grant of the patent:
**27.09.2006 Bulletin 2006/39**

(21) Application number: **03792390.1**

(22) Date of filing: **19.08.2003**

(51) Int Cl.:
*C07D 239/42* (2006.01) *C07D 401/12* (2006.01)
*A61K 31/505* (2006.01) *A61K 31/506* (2006.01)
*A61P 25/06* (2006.01)

(86) International application number:
**PCT/EP2003/009221**

(87) International publication number:
**WO 2004/018434 (04.03.2004 Gazette 2004/10)**

(54) **2-PHENYLAMINO-4-TRIFLUOROMETHYL-5-(BENZYL- OR PYRIDIN-4-YLMETHYL) CARBAMOYLPYRIMIDINE DERIVATIVES AS SELECTIVE CB2 CANNABINOID RECEPTOR MODULATORS**

2-PHENYLAMINO-4-TRIFLUOROMETHYL-5-(BENZYL- ODER PYRIDIN-4-YLMETHYL) CARBAMOYLPYRIMIDIN-DERIVATE ALS SELEKTIVE CB2 CANNABINOID-REZEPTOR MODULATOREN

DERIVES DE 2-PHENYLAMINO-4-TRIFLUOROMETHYL-5-(BENZYL- OU PYRIDIN-4-YLMETHYL) CARBAMOYLPYRIMIDINE COMME MODULATEURS SELECTIFS DU RECEPTEUR CANNABINOIDE CB2

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **21.08.2002 GB 0219500**
**11.07.2003 GB 0316332**

(43) Date of publication of application:
**01.06.2005 Bulletin 2005/22**

(73) Proprietor: **GLAXO GROUP LIMITED**
**Greenford, Middlesex UB6 ONN (GB)**

(72) Inventors:
- **EATHERTON, Andrew John, GlaxoSmithKline Welwyn, Hertfordshire AL6 9AR (GB)**
- **GIBLIN, Gerard Martin Paul, GlaxoSmithKline Welwyn, Hertfordshire AL6 9AR (GB)**
- **GREEN, Richard H. Deceased (GB)**
- **MITCHELL, William Leonard, GlaxoSmithKline Welwyn, Hertfordshire AL6 9AR (GB)**
- **NAYLOR, Alan, GlaxoSmithKline Stevenage, Hertfordshire SG1 2NY (GB)**
- **RAWLINGS, Derek Anthony, GlaxoSmithKline Welwyn, Hertfordshire AL6 9AR (GB)**
- **SLINGSBY, Brian Peter, GlaxoSmithKline Welwyn, Hertfordshire AL6 9AR (GB)**
- **WHITTINGTON, Andrew Richard, GlaxoSmithKline Stevenage, Hertfordshire SG1 2NY (GB)**
- **DOUGHTY, Jennifer, Margaret Portland, ME 04103 (US)**

(74) Representative: **Billson, Siân Catherine et al**
**GlaxoSmithKline**
**Corporate Intellectual Property (CN9.25.1)**
**980 Great West Road**
**Brentford, Middlesex TW8 9GS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

(56) References cited:
WO-A-97/09315
WO-A-02/066036

## Description

The present invention relates to novel pyrimidine derivatives, pharmaceutical compositions containing these compounds and their use in the treatment of diseases, particularly pain, which diseases are caused directly or indirectly by an increase or decrease in activity of the cannabinoid receptor.

Cannabinoids are a specific class of psychoactive compounds present in Indian cannabis (*Cannabis sativa*), including about sixty different molecules, the most representative being cannabinol, cannabidiol and several isomers of tetrahydrocannabinol. Knowledge of the therapeutic activity of cannabis dates back to the ancient dynasties of China, where, 5,000 years ago, cannabis was used for the treatment of asthma, migraine and some gynaecological disorders. These uses later became so established that, around 1850, cannabis extracts were included in the US Pharmacopaeia and remained there until 1947.

Cannabinoids are known to cause different effects on various systems and/or organs, the most important being on the central nervous system and on the cardiovascular system. These effects include alterations in memory and cognition, euphoria, and sedation. Cannabinoids also increase heart rate and vary systemic arterial pressure. Peripheral effects related to bronchial constriction, immunomodulation, and inflammation have also been observed. The capability of cannabinoids to reduce intraocular pressure and to affect respiratory and endocrine systems is also well documented. See e.g. L.E. Hollister, Health Aspects of Cannabis, Pharmacological Reviews, Vol. 38, pp. 1-20, (1986). More recently, it was found that cannabinoids suppress the cellular and humoral immune responses and exhibit antiinflammatory properties. Wirth et al., Antiinflammatory Properties of Cannabichrome, Life Science, Vol. 26, pp. 1991-1995, (1980).

In spite of the foregoing benefits, the therapeutic use of cannabis is controversial, both due to its relevant psychoactive effects (causing dependence and addiction), and due to manifold side effects that have not yet been completely clarified. Although work in this field has been ongoing since the 1940's, evidence indicating that the peripheral effects of cannabinoids are directly mediated, and not secondary to a CNS effect, has been limited by the lack of receptor characterization, the lack of information concerning an endogenous cannabinoid ligand and, until recently, the lack of receptor subtype selective compounds.

The first cannabinoid receptor was found to be mainly located in the brain, in neural cell lines, and, only to a lesser extent, at the peripheral level. In view of its location, it was called the central receptor ("CB 1 "). See Matsuda et al., "Structure of a Cannabinoid Receptor and Functional Expression of the Cloned cDNA," Nature, Vol. 346, pp. 561-564 (1990. The second cannabinoid receptor ("CB2") was identified in the spleen, and was assumed to modulate the non psychoactive effects of the cannabinoids. See Munro et el., "Molecular Characterization of a Peripheral Receptor for Cannabinoids," Nature, Vol. 365, pp. 61-65 (1993).

Recently, some compounds have been prepared which are capable of acting as agonists on both the cannabinoid receptors. For example, use of derivatives of dihydroxypyrrole-(1,2,3-d,e)-1,4-benzoxazine in the treatment of glaucoma and the use of derivatives of 1,5-diphenylpyrazole as immunomodulators or psychotropic agents in the treatment of various neuropathologies, migraine, epilepsy, glaucoma, etc are known. See U.S. Patent No. 5,112,820 and EP 576357, respectively. However, because these compounds are active on both the CB 1 and CB2 receptor, they can lead to serious psychoactive effects.

The foregoing indications and the preferential localization of the CB2 receptor in the immune system confirms a specific role of CB2 in modulating the immune and antiinflammatory response to stimuli of different sources.

The total size of the patient population suffering from pain is vast (almost 300 million), dominated by those suffering from back pain, osteo-arthritic pain and post-operative pain. Neuropathic pain (associated with neuronal lesions such as those induced by diabetes, HIV, herpes infection, or stroke) occurs with lower, but still substantial prevalence, as does cancer pain.

The pathogenic mechanisms that give rise to pain symptoms can be grouped into two main categories:

- those that are components of inflammatory tissue responses (Inflammatory Pain):
- those that result from a neuronal lesion of some form (Neuropathic Pain).

Chronic inflammatory pain consists predominantly of osteoarthritis, chronic low back pain and rheumatoid arthritis. The pain results from acute and on-going injury and/or inflammation. There may be both spontaneous and provoked pain.

There is an underlying pathological hypersensitivity as a result of physiological hyperexcitability and the release of inflammatory mediators which further potentiate this hyperexcitability. CB2 receptors are expressed on inflammatory cells (T cells, B cells, macrophages, mast cells) and mediate immune suppression through inhibition of cellular interaction/ inflammatory mediator release. CB2 receptors may also be expressed on sensory nerve terminals and therefore directly inhibit hyperalgesia.

The role of CB2 in immunomodulation, inflammation, osteoporosis, cardiovascular, renal and other disease

conditions is now being examined. In light of the fact that cannabinoids act on receptors capable of modulating different functional effects, and in view of the low homology between CB2 and CB1, the importance of developing a class of drugs selective for the specific receptor sub-type is evident. The natural or synthetic cannabinoids currently available do not fulfil this function because they are active on both receptors.

Based on the foregoing, there is a need for compounds which are capable of selectively modulating the receptor for cannabinoids and, therefore, the pathologies associated with such receptors. Thus, CB2 modulators offer a unique approach toward the pharmacotherapy of immune disorders, inflammation, osteoporosis, renal ischemia and other pathophysiological conditions.

The present invention provides novel pyrimidine derivatives of formula (I), pharmaceutical compositions containing these compounds, and their use as CB2 receptor modulators, which are useful in the treatment of a variety of disorders.

The invention provides compounds of formula (I)

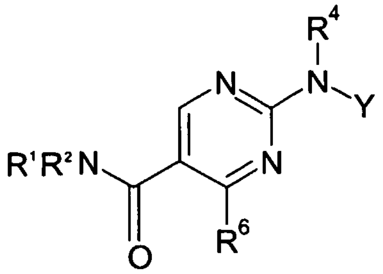

selected from:

2-(3-Chlorophenylamino)-4-trifluoromethylpyrimidine-5-carboxylic acid benzyl-amide; 2-(3-Chlorophenylamino)-4-trifluoromethylpyrimidine-5-carboxylic acid (pyridin-4-ylmethyl)amide;
2-(3-Chlorophenylamino)-4-trifluoromethylpyrimidine-5-carboxylic acid N-benzyl-N-methylamide;
2-(3-Chlorophenylamino)-4-trifluoromethylpyrimidine-5-carboxylic acid 4-methoxybenzyl-amide;
2-(3-Chlorophenylamino)-4-trifluoromethylpyrimidine-5-carboxylic acid 4-fluorobenzyl-amide;
2-(3-Chlorophenylamino)-4-trifluoromethylpyrimidine-5-carboxylic acid 4-cyanobenzyl-amide;
2-(2,3-Dichlorophenylamino)-4-trifluoromethylpyrimidine-5-carboxylic acid N-benzyl-N-methylamide;
2-(2,4-Dichlorophenylamino)-4-trifluoromethylpyrimidine-5-carboxylic acid benzyl-amide;
2-(3,4-Dichlorophenylamino)-4-trifluoromethylpyrimidine-5-carboxylic acid benzyl-amide;
2-(2,6-Dichlorophenylamino)-4-trifluoromethylpyrimidine-5-carboxylic acid N-benzyl-N-methylamide;
2-(3,5-Dichlorophenylamino)-4-trifluoromethylpyrimidine-5-carboxylic acid benzyl-amide;
2-(3-Fluorophenylamino)-4-trifluoromethylpyrimidine-5-carboxylic acid benzyl-amide;
2-(3-Bromophenylamino)-4-trifluoromethylpyrimidine-5-carboxylic acid benzyl-amide;
2-(3-Bromophenylamino)-4-trifluoromethylpyrimidine-5-carboxylic acid N-benzyl-N-methylamide;
2-(2-Methoxyphenylamino)-4-trifluoromethylpyrimidine-5-carboxylic acid benzyl-amide;
2-(2,3-Dichlorophenylamino)-4-trifluoromethylpyrimidine-5-carboxylic acid (pyridin-4-ylmethyl)amide;
2-(2,4-Dichlorophenylamino)-4-trifluoromethylpyrimidine-5-carboxylic acid (pyridin-4-ylmethyl)amide;
2-(3,4-Dichlorophenylamino)-4-trifluoromethylpyrimidine-5-carboxylic acid (pyridin-4-ylmethyl)amide;
2-(2,5-Dichlorophenylamino)-4-trifluoromethylpyrimidine-5-carboxylic acid (pyridin-4-ylmethyl)amide;
2-(3-Fluorophenylamino)-4-trifluoromethylpyrimidine-5-carboxylic acid (pyridin-4-ylmethyl)amide;
2-(3-Bromophenylamino)-4-trifluoromethylpyrimidine-5-carboxylic acid (pyridin-4-ylmethyl)amide;
2-(3,5-Dichlorophenylamino)-4-trifluoromethylpyrimidine-5-carboxylic acid (pyridin-4-ylmethyl)amide;
2-(3-Fluorophenylamino)-4-trifluoromethylpyrimidine-5-carboxylic acid 4-fluorobenzyl-amide; 2-(3-Bromophenylamino)-4-trifluoromethylpyrimidine-5-carboxylic acid 4-fluorobenzyl-amide;
2-(3,5-Dichlorophenylamino)-4-trifluoromethylpyrimidine-5-carboxylic acid 4-fluorobenzylamide;
2-(3,5-Dichlorophenylamino)-4-trifluoromethylpyrimidine-5-carboxylic acid 4-cyanobenzyl-amide;
2-(4-Cyano-phenylamino)-4-trifluoromethyl-pyrimidine-5-carboxylic acid benzylamide;
2-(3-Chloro-phenylamino)-4-trifluoromethyl-pyrimidine-5-carboxylic acid (2-methyl-pyridin-4-ylmethyl)-amide hydrochloride;
2-(3-Chloro-phenylamino)-4-trifluoromethyl-pyrimidine-5-carboxylic acid (2-fluoro-pyridin-4-ylmethyl)-amide;

and pharmaceutically acceptable salts or solvates thereof.

We have found that at least in the CB2 assay described herein the following compounds are inactive;
2-(4-*tert*-butyl-phenylamino)-4-trifluoromethyl-pyrimidine-5-carboxylic acid benzylamide;
2-(4-*tert*-butyl-phenylamino)-4-trifluoromethyl-pyrimidine-5-carboxylic acid benzyl-methylamide;
2-(3-Chloro-phenylamino)-4-trifluoromethyl-pyrimidine-5-carboxylic acid 2-methoxy-benzylamide;
2-(3-Chloro-phenylamino)-4-trifluoromethyl-pyrimidine-5-carboxylic acid 2-bromo-benzylamide.

In one particular embodiment the compounds are selective for CB2 over CB1. Preferably the compounds are 100 fold selective i.e. compounds of formula (I) have an EC50 value at the cloned human cannabinoid CB2 receptor of at least 100 times the EC50 values at the cloned humna cannabinoid CB receptor or have less than 10% efficacy at the CB1 receptor.

It will be appreciated that, for pharmaceutical use, the salts referred to above will be physiologically acceptable salts, but other salts may find use, for example in the preparation of compounds of formula (I) and the physiological acceptable salts thereof. Pharmaceutically acceptable salts include those described by Berge, Bighley and Monkhouse , J. Pharm. Sci., 1977, 66, 1-19. The term "pharmaceutically acceptable salts" refers to salts prepared from pharmaceutically acceptable non-toxic bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropyl amine, tromethamine, and the like. When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Preferred examples of pharmaceutically acceptable salts include the ammonium, calcium, magnesium, potassium, and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicylic, citric, gluconic, aspartic, stearic, palmitic, itaconic, glycolic, p-aminobenzoic, glutamic, benzenesulfonic, cyclohexylsulfamic, phosphoric and nitric acids.

Compounds of formula (I) can be prepared as set forth in the following scheme:

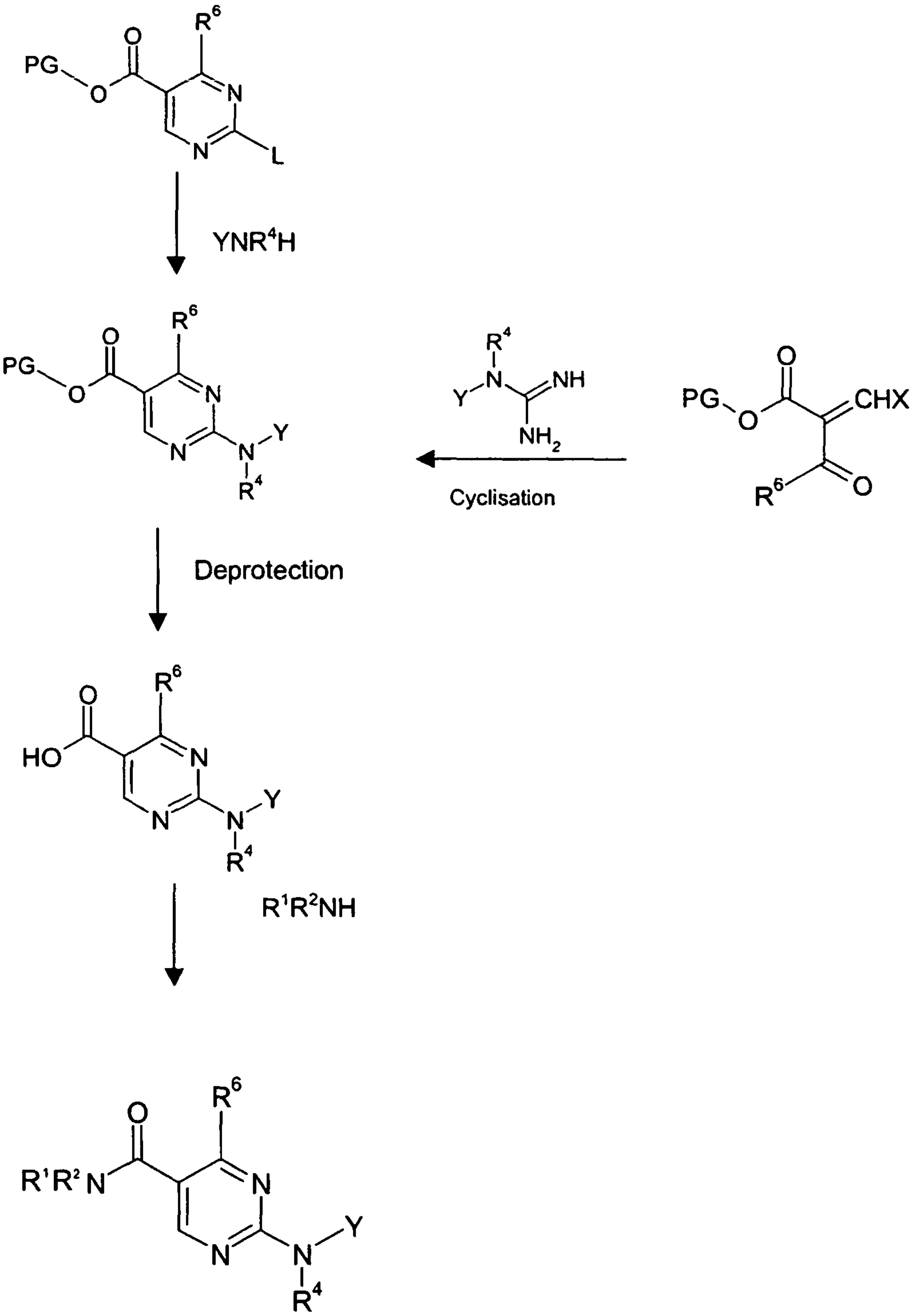


wherein L is a leaving group, for example halo, PG is a protecting group for example methyl, ethyl or benzyl, X is a leaving group, for example halo, $OC_{1-6}$ alkyl eg O-methyl or O-ethyl or $NR^cR^d$ wherein $R^c$ and $R^d$ are independently selected from $C_{1-6}$ alkyl eg methyl and $R^1$, $R^2$, $R^4$, $R^6$ and Y are as defined for compounds of formula (I).

It is to be understood that the present invention encompasses all isomers of compounds of formula (I), that is all geometric, tautomeric and optical forms, and mixtures thereof (e.g. racemic mixtures). Where additional chiral centres are present in compounds of formula (I), the present invention includes within its scope all possible diastereoismers, including mixtures thereof. The different isomeric forms may be separated or resolved one from the other by conventional methods, or any given isomer may be obtained by conventional synthetic methods or by stereospecific or asymmetric syntheses. The subject invention also includes isotopically-labeled compounds, which are identical to those recited in

formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as $^3H$, $^{11}C$, $^{14}C$, $^{18}F$, $^{123}I$ and $^{125}I$.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as $^3H$, $^{14}C$ are incorporated, are useful in drug and/or substrate tissue distribution assays. Tritiated, i.e., $^3H$, and carbon-14, i.e., $^{14}C$, isotopes are particularly preferred for their ease of preparation and detectability. $^{11}C$ and $^8F$ isotopes are particularly useful in PET (positron emission tomography), and $^{125}I$ isotopes are particularly useful in SPECT (single photon emission computerized tomography), all useful in brain imaging. Further, substitution with heavier isotopes such as deuterium, i.e., $^2H$, can afford certain therapeutic advantages resulting from greater metabolic stability, for example increased in vivo half-life or reduced dosage requirements and, hence, may be preferred in some circumstances. Isotopically labeled compounds of formula I and following of this invention can generally be prepared by carrying out the procedures disclosed in the Schemes and/or in the Examples below, by substituting a readily available isotopically labeled reagent for a non-isotopically labeled reagent.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

The compounds of the invention bind selectively to the CB2 receptor, and are therefore useful in treating CB2 receptor mediated diseases.

In view of their ability to bind to the CB2 receptor, the compounds of the invention may be useful in the treatment of the disorders that follow. Thus, the compounds of formula (I) may be useful as analgesics. For example they may be useful in the treatment of chronic inflammatory pain (e.g. pain associated with rheumatoid arthritis, osteoarthritis, rheumatoid spondylitis, gouty arthritis and juvenile arthritis) including the property of disease modification and joint structure preservation; musculoskeletal pain; lower back and neck pain; sprains and strains; neuropathic pain; sympathetically maintained pain; myositis; pain associated with cancer and fibromyalgia; pain associated with migraine; pain associated with influenza or other viral infections, such as the common cold; rheumatic fever; pain associated with functional bowel disorders such as non-ulcer dyspepsia, non-cardiac chest pain and irritable bowel syndrome; pain associated with myocardial ischemia; post operative pain; headache; toothache; and dysmenorrhea.

The compounds of the invention may also be useful disease modification or joint structure preservation in multiple sclerosis, rheumatoid arthritis, osteo-arthritis, rheumatoid spondylitis, gouty arthritis and juvenile arthritis.

The compounds of the invention may be particularly useful in the treatment of neuropathic pain. Neuropathic pain syndromes can develop following neuronal injury and the resulting pain may persist for months or years, even after the original injury has healed. Neuronal injury may occur in the peripheral nerves, dorsal roots, spinal cord or certain regions in the brain. Neuropathic pain syndromes are traditionally classified according to the disease or event that precipitated them. Neuropathic pain syndromes include: diabetic neuropathy; sciatica; nonspecific lower back pain; multiple sclerosis pain; fibromyalgia; HIV-related neuropathy; post-herpetic neuralgia; trigeminal neuralgia; and pain resulting from physical trauma, amputation, cancer, toxins or chronic inflammatory conditions. These conditions are difficult to treat and although several drugs are known to have limited efficacy, complete pain control is rarely achieved. The symptoms of neuropathic pain are incredibly heterogeneous and are often described as spontaneous shooting and lancinating pain, or ongoing, burning pain. In addition, there is pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia).

The compounds of formula (I) may also be useful in the treatment of fever.

The compounds of formula (I) may also be useful in the treatment of inflammation, for example in the treatment of skin conditions (e.g. sunburn, bums, eczema, dermatitis, psoriasis); ophthalmic diseases such as glaucoma, retinitis, retinopathies, uveitis and of acute injury to the eye tissue (e.g. conjunctivitis); lung disorders (e.g. asthma, bronchitis, emphysema, allergic rhinitis, respiratory distress syndrome, pigeon fancier's disease, farmer's lung, chronic obstructive pulmonary disease, (COPD); gastrointestinal tract disorders (e.g. aphthous ulcer, Crohn's disease, atopic gastritis, gastritis varialoforme, ulcerative colitis, coeliac disease, regional ileitis, irritable bowel syndrome, inflammatory bowel disease, gastrointestinal reflux disease); organ transplantation; other conditions with an inflammatory component such as vascular disease, migraine, periarteritis nodosa, thyroiditis, aplastic anaemia, Hodgkin's disease, sclerodoma, myaesthenia gravis, multiple sclerosis, sorcoidosis, nephrotic syndrome, Bechet's syndrome, polymyositis, gingivitis, myocardial ischemia, pyrexia, systemic lupus erythematosus, tendinitis, bursitis, and Sjogren's syndrome.

The compounds of formula (I) are also useful in the treatment of immunological diseases such as autoimmune

diseases, immunological deficiency diseases or organ transplantation. The compounds of formula (I) are also effective in increasing the latency of HIV infection.

The compounds of formula (I) are also useful in the treatment of diseases of abnormal platelet function (e.g. occlusive vascular diseases).

The compounds of formula (I) are also useful for the preparation of a drug with diuretic action.

The compounds of formula (I) are also useful in the treatment of impotence or erectile dysfunction.

The compounds of formula (1) are also useful for attenuating the hemodynamic side effects of non-steroidal anti-inflammatory drugs (NSAm's) and cyclooxygenase-2 (COX-2) inhibitors.

The compounds of formula (I) are also useful in the treatment of neurodegenerative diseases and neurodegeneration such as dementia, particularly degenerative dementia (including senile dementia, Alzheimer's disease, Pick's disease, Huntingdon's chorea, Parkinson's disease and Creutzfeldt-Jakob disease, motor neuron disease); vascular dementia (including multi-infarct dementia); as well as dementia associated with intracranial space occupying lesions; trauma; infections and related conditions (including HIV infection); dementia in Parkinson's disease, metabolism; toxins; anoxia and vitamin deficiency; and mild cognitive impairment associated with ageing, particularly Age Associated Memory Impairment. The compounds may also be useful for the treatment of amyotrophic lateral sclerosis (ALS) and neuroinflamation.

The compounds of formula (I) are also useful in neuroprotection and in the treatment of neurodegeneration following stroke, cardiac arrest, pulmonary bypass, traumatic brain injury, spinal cord injury or the like.

The compounds of formula (I) are also useful in the treatment of tinnitus.

The compounds of formula (I) are also useful in the treatment of psychiatric disease for example schizophrenia, depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, schizoaffective disorder or the depressed type, and depressive disorders resulting from general medical conditions including, but not limited to, myocardial infarction, diabetes, miscarriage or abortion, *etc*), anxiety disorders (including generalised anxiety disorder and social anxiety disorder), panic disorder, agoraphobia, social phobia, obsessive compulsive disorder and post-traumatic stress disorder, memory disorders, including dementia, amnesic disorders and age-associated memory impairment, disorders of eating behaviours, including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), amphetamine or amphetamine-related drugs (e.g. dextroamphetamine, methylamphetamine) or a combination thereof.

The compounds of formula (I) are also useful in preventing or reducing dependence on, or preventing or reducing tolerance or reverse tolerance to, a dependence - inducing agent. Examples of dependence inducing agents include opioids (e.g. morphine), CNS depressants (e.g. ethanol), psychostimulants (e.g. cocaine) and nicotine.

The compounds of formula (I) are also useful in the treatment of kidney dysfunction (nephritis, particularly mesangial proliferative glomerulonephritis, nephritic syndrome), liver dysfunction (hepatitis, cirrhosis), gastrointestinal dysfunction (diarrhoea) and colon cancer.

It is to be understood that references to treatment includes both treatment of established symptoms and prophylactic treatment unless explicitly stated otherwise.

According to a further aspect of the invention, we provide a compound of formula (I) or a pharmaceutically acceptable salt or solvate thereof for use in human or veterinary medicine.

According to another aspect of the invention, we provide a compound of formula (I) or a pharmaceutically acceptable salt or solvate thereof for use in the treatment of a condition which is mediated by the activity of cannabinoid 2 receptors.

According to a further aspect of the invention, we provide a method of treating a human or animal subject suffering from a condition which is mediated by the activity of cannabinoid 2 receptors which comprises administering to said subject a therapeutically effective amount of a compound of formula (I) or a pharmaceutically acceptable salt or solvate thereof.

According to a further aspect of the invention we provide a method of treating a human or animal subject suffering from an immune disorder, an inflammatory disorder, pain, rheumatoid arthritis, multiple sclerosis, osteoarthritis or osteoporosis which method comprises administering to said subject an effective amount of a compound of formula (I) or a pharmaceutically acceptable salt or solvate thereof.

Preferably the pain is selected from inflammatory pain, viseral pain, cancer pain, neuropathic pain, lower back pain, muscular sceletal, post operative pain, acute pain and migraine. More preferably the inflammatory pain is pain associated with rheumatoid arthritis or osteoarthritis.

According to another aspect of the invention is provided the use of a compound of formula (I) or a pharmaceutically acceptable salt or solvate thereof for the manufacture of a therapeutic agent for the treatment or prevention of a condition such an immune disorder, an inflammatory disorder, pain, rheumatoid arthritis, multiple sclerosis, osteoarthritis or osteoporosis which method comprises administering to said subject an effective amount of a compound of formula (I) or a pharmaceutically acceptable salt or solvate thereof.

In order to use a compound of formula (I) or a pharmaceutically acceptable salt or solvate thereof for the treatment of humans and other mammals it is normally formulated in accordance with standard pharmaceutical practice as a pharmaceutical composition. Therefore in another aspect of the invention is provided a pharmaceutical composition comprising a compound of formula (I) or a pharmaceutically acceptable salt or solvate thereof adapted for use in human or veterinary medicine.

As used herein, "modulator" means both antagonist, partial agonist and a full agonist, and inverse agonist. Preferably the present modulators are agonists.

Compounds of formula (I) and their pharmaceutically acceptable salts or solvates may be administered in a standard manner for the treatment of the indicated diseases, for example orally, parentarally, sub-lingually, dermally, intranasally, transdermally, rectally, via inhalation or via buccal administration.

Compositions of formula (I) and their pharmaceutically acceptable salts or solvates which are active when given orally can be formulated as syrups, tablets, capsules and lozenges. A syrup formulation will generally consist of a suspension or solution of the compound or salt in a liquid carrier for example, ethanol, peanut oil, olive oil, glycerine or water with a flavouring or colouring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound in a sterile aqueous or non-aqueous carrier optionally containing a parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of formula (I) or a pharmaceutically acceptable salt or solvate thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit for oral administration contains suitably from 0.1 mg to 500 mg/Kg, and preferably from 1 mg to 100 mg/Kg, and each dosage unit for parenteral administration contains suitably from 0.1 mg to 100 mg/Kg, of a compound of formula(I) or a pharmaceutically acceptable salt or solvate thereof calculated as the free acid. Each dosage unit for intranasal administration contains suitably 1-400 mg and preferably 10 to 200 mg per person. A topical formulation contains suitably 0.01 to 5.0% of a compound of formula (I).

The daily dosage regimen for oral administration is suitably about 0.01 mg/Kg to 40 mg/Kg, of a compound of formula(I) or a pharmaceutically acceptable salt or solvate thereof calculated as the free acid. The daily dosage regimen for parenteral administration is suitably about 0.001 mg/Kg to 40 mg/Kg, of a compound of formula (I) or a pharmaceutically acceptable salt or solvate thereof calculated as the free acid. The daily dosage regimen for intranasal administration and oral inhalation is suitably about 10 to about 500 mg/person. The active ingredient may be administered from 1 to 6 times a day, sufficient to exhibit the desired activity.

It may be advantageous to prepare the compounds of the present invention as nanoparticles. This may improve the oral bioavailability of the compounds. For the purposes of the present invention "nanoparticulate" is defined as solid particles with 50% of the particles having a particle size of less than 1 $\mu$m, more preferably less than 0.75$\mu$m

The particle size of the solid particles of compound (I) may be determined by laser diffraction. A suitable machine for determining particle size by laser diffraction is a Lecotrac laser particle size analyser, using an HELOS optical bench fitted with a QUIXEL dispersion unit.

Numerous processes for the synthesis of solid particles in nanoparticulate form are known. Typically these processes involve a milling process, preferably a wet milling process in the presence of a surface modifying agent that inhibits aggregation and/or crystal growth of the nanoparticles once created. Alternatively these processes may involve a precipitation process, preferably a process of precipitation in an aqueous medium from a solution of the drug in a non-aqueous solvent.

Accordingly, in a further aspect, the present invention provides a process for preparing compound (I) in nanoparticulate form as hereinbefore defined, which process comprises milling or precipitation.

Representative processes for the preparation of solid particles in nanoparticulate form are described in the patents and publications listed below.

U.S. Patent No. 4,826,689 to Violanto & Fischer, U. S. Patent No. 5,145,684 to Liversidge et al U.S Patent No. 5,298,262 to Na & Rajagopalan, U.S. Patent No. 5,302,401 Liversidge et al U.S. Patent No. 5,336,507 to Na & Rajagopalan, U.S. Patent No. 5,340,564 to Illig & Sarpotdar U.S. Patent No. 5,346,702 to Na Rajagopalan, U.S. Patent No. 5,352,459 to Hollister et al U.S. Patent No. 5,354,560 to Lovrecich, U.S. Patent No. 5,384,124 to Courteille et al, U.S. Patent No. 5,429,824 to June, U.S. Patent No. 5,503,723 to Ruddy et al, U.S. Patent No. 5,510 118 to Bosch et al, U.S. Patent No. 5,518 to Bruno et al, U.S. Patent No. 5,518,738 to Eickhoff et al, U.S. Patent No. 5,534,270 to De Castro, U.S. Patent No. 5,536,508 to Canal et al, U.S. Patent No. 5,552,160 to Liversidge et al, U.S. Patent No. 5,560,931 to Eickhoff et al, U.S. Patent No. 5,560,932 to Bagchi et al, U.S. Patent No. 5,565,188 to Wong et al, U.S. Patent No. 5,571,536 to Eickhoff et al, U.S. Patent No. 5,573,783 to Desieno & Stetsko, U.S Patent No. 5,580,579 to Ruddy et al, U.S. Patent No 5,585,108 to Ruddy et al, U.S. Patent No. 5,587,143 to Wong, U.S. Patent No. 5,591456 to Franson et al, U.S. Patent No. 5,622,938 to Wong, U.S. Patent No 5,662,883 to Bagchi et al, U.S. Patent No. 5,665,331 to Bagchi et al, U.S Patent No. 5,718,919 to Ruddy et al, U.S. Patent No. 5,747,001 to Wiedmann et al, WO93/25190, WO96/24336, WO 97/14407, WO 98/35666, WO 99/65469, WO 00/18374, WO 00/27369, WO 00/30615 and WO 01/41760.

Such processes may be readily adapted for the preparation of compound (I) in nanoparticulate form. Such processes form a further aspect of the invention.

The process of the present invention preferably uses a wet milling step carried out in a mill such as a dispersion mill in order to produce a nanoparticulate form of the compound. The present invention may be put into practice using a conventional wet milling technique, such as that described in Lachman *et al.,* The Theory and Practice of Industrial Pharmacy, Chapter 2, "Milling" p.45 (1986).

In a further refinement, PCT/EP01/07085 (SmithKline Beecham plc) describes a wet milling procedure using a mill in which at least some of the surfaces are made of nylon (polyamide) comprising one or more internal lubricants, for use in the preparation of solid particles of a drug substance in nanoparticulate form.

In another aspect the present invention provides a process for preparing compounds of the invention in nanoparticulate form comprising wet milling a suspension of compound in a mill having at least one chamber and agitation means, said chamber(s) and/or said agitation means comprising a lubricated nylon, as described in PCT/EP01/07085.

The suspension of a compound of the invention for use in the wet milling is typically a liquid suspension of the coarse compound in a liquid medium. By "suspension" is meant that the compound is essentially insoluble in the liquid medium. Representative liquid media include an aqueous medium. Using the process of the present invention the average particle size of coarse compound of the invention may be up to 1mm in diameter. This advantageously avoids the need to pre-process the compound.

In a further aspect of the invention the aqueous medium to be subjected to the milling comprises compound (I) present in from about 1% to about 40% w/w, preferably from about 10% to about 30% w/w, more preferably about 20% w/w.

The aqueous medium may further comprise one or more pharmaceutically acceptable watersoluble carriers which are suitable for steric stabilisation and the subsequent processing of compound (I) after milling to a pharmaceutical composition, e.g. by spray drying. Pharmaceutically acceptable excipients most suitable for steric stabilisation and spray-drying are surfactants such as poloxamers, sodium lauryl sulphate and polysorbates etc; stabilisers such as celluloses e.g. hydroxypropylmethyl cellulose; and carriers such as carbohydrates e.g. mannitol.

In a further aspect of the invention the aqueous medium to be subjected to the milling may further comprise hydroxypropylmethyl cellulose (HPMC) present in from about 0.1 to about 10% w/w.

The process of the present invention may comprise the subsequent step of drying compound of the invention to yield a powder.

Accordingly, in a further aspect, the present invention provides a process for preparing a pharmaceutical composition contain a compound of the present invention which process comprises producing compound of formula (I) in nanoparticulate form optionally followed by drying to yield a powder.

A further aspect of the invention is a pharmaceutical composition comprising a compound of formula (I) or a pharmaceutically acceptable salt or solvate thereof in which the compound of formula (I) or a pharmaceutically acceptable salt or solvate thereof is present in solid particles in nanoparticulate form, in admixture with one or more pharmaceutically acceptable carriers or excipients.

By "drying" is meant the removal of any water or other liquid vehicle used during the process to keep compound of formula (I) in liquid suspension or solution. This drying step may be any process for drying known in the art, including freeze drying, spray granulation or spray drying. Of these methods spray drying is particularly preferred. All of these techniques are well known in the art. Spray drying/fluid bed granulation of milled compositions is carried out most suitably

using a spray dryer such as a Mobile Minor Spray Dryer [Niro, Denmark], or a fluid bed drier, such as those manufactured by Glatt, Germany.

In a further aspect the invention provides a pharmaceutical composition as hereinbefore defined, in the form of a dried powder, obtainable by wet milling solid particles of compound of formula (I) followed by spray-drying the resultant suspension.

Preferably, the pharmaceutical composition as hereinbefore defined, further comprises HPMC present in less than 15% w/w, preferably in the range 0.1 to 10% w/w.

The $CB_2$ receptor compounds for use in the instant invention may be used in combination with other therapeutic agents, for example COX-2 inhibitors, such as celecoxib, deracoxib, rofecoxib, valdecoxib, parecoxib or COX-189; 5-lipoxygenase inhibitors; NSAID's, such as aspirin, diclofenac, indomethacin, nabumetone or ibuprofen; leukotriene receptor antagonists; DMARD's such as methotrexate; adenosine A1 receptor agonists; sodium channel blockers, such as lamotrigine; NMDA receptor modulators, such as glycine receptor antagonists; gabapentin and related compounds; tricyclic antidepressants such as amitriptyline; neurone stabilising antiepileptic drugs; mono-aminergic uptake inhibitors such as venlafaxine; opioid analgesics; local anaesthetics; $5HT_1$ agonists, such as triptans, for example sumatriptan, naratriptan, zolmitriptan, eletriptan, frovatriptan, almotriptan or rizatriptan; $EP_1$ receptor ligands, $EP_4$ receptor ligands; $EP_2$ receptor ligands; $EP_3$ receptor ligands; $EP_4$ antagonists; $EP_2$ antagonists and $EP_3$ antagonists; bradykinin receptor ligands and vanilloid receptor ligand. antirheumatoid arthritis drugs, for example anti TNF drugs e.g. enbrel, remicade, anti-IL-1 drugs, or DMARDS e.g. leflunamide. When the compounds are used in combination with other therapeutic agents, the compounds may be administered either sequentially or simultaneously by any convenient route.

Additional COX-2 inhibitors are disclosed in US Patent Nos. 5,474,995 US5,633,272; US5,466,823, US6,310,099 and US6,291,523; and in WO 96/25405, WO 97/38986, WO 98/03484, WO 97/14691, WO99/12930, WO00/26216, WO00/52008, WO00/38311, WO01/58881 and WO02/18374.

The invention thus provides, in a further aspect, a combination comprising a compound of formula (I) or a pharmaceutically acceptable salt or solvate thereof together with a further therapeutic agent or agents.
Suitable 5HT3 antagonists which may be used in combination of the compound of the inventions include for example ondansetron, granisetron, metoclopramide.

Suitable serotonin agonists which may be used in combination with the compound of the invention include sumatriptan, rauwolscine, yohimbine, metoclopramide.

Suitable SSRIs which may be used in combination with the compound of the invention include fluoxetine, citalopram, femoxetine, fluvoxamine, paroxetine, indalpine, sertraline, zimeldine.

Suitable SNRIs which may be used in combination with the compound of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of any of the above combinations or compositions may be administered simultaneously (either in the same or different pharmaceutical formulations), separately or sequentially.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a pharmaceutically acceptable salt or solvate thereof is used in combination with a second therapeutic agent active against the same disease state the dose of each compound may differ from that when the compound is used alone. Appropriate doses will be readily appreciated by those skilled in the art.

**Determination of cannabinoid CB1 Receptor Agonist Activity**

The cannabinoid CB 1 receptor agonist activity of the compounds of formula (I) was determined in accordance with the following experimental method.

**Experimental Method**

Yeast (*Saccharomyces cerevisiae*) cells expressing the human cannabinoid CB 1 receptor were generated by integration of an expression cassette into the *ura3* chromosomal locus of yeast strain MMY23. This cassette consisted of DNA sequence encoding the human CB1 receptor flanked by the yeast GPD promoter to the 5' end of CB 1 and a yeast transcriptional terminator sequence to the 3' end of CB1. MMY23 expresses a yeast/mammalian chimeric G-protein alpha subunit in which the C-terminal 5 amino acids ofGpal are replaced with the C-terminal 5 amino acids of human Gαi3 (as described in Brown *et al.* (2000), *Yeast* **16**:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1M sodium phosphate pH 7.0, and 20μM fluorescein di-β-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.

Compounds of the Examples tested according to this method had $EC_{50}$ values >2000nM and/or efficacy values of <50% at the cloned human cannabinoid CB1 receptor.

**Determination of cannabinoid CB2 Receptor Agonist Activity**

The cannabinoid CB2 receptor agonist activity of the compounds of formula (I) was determined in accordance with the following experimental method.

**Experimental Method**

Yeast (*Saccharomyces cerevisiae*) cells expressing the human cannabinoid CB2 receptor were generated by integration of an expression cassette into the *ura3* chromosomal locus of yeast strain MMY23. This cassette consisted of DNA sequence encoding the human CB2 receptor flanked by the yeast GPD promoter to the 5' end of CB2 and a yeast transcriptional terminator sequence to the 3' end of CB2. MMY23 expresses a yeast/mammalian chimeric G-protein alpha subunit in which the C-terminal 5 amino acids of Gpa1 are replaced with the C-terminal 5 amino acids of human Gαi3 (as described in Brown *et al.* (2000), *Yeast* **16**:11-22). Cells were grown at 30°C in liquid Synthetic Complete (SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM stocks in DMSO. $EC_{50}$ values (the concentration required to produce 50% maximal response) were estimated using dilutions of between 3- and 5-fold (BiomekFX, Beckman) into DMSO. Agonist solutions in DMSO (1% final assay volume) were transferred into black, clear bottom, microtitre plates from NUNC (96- or 384-well). Cells were suspended at a density of 0.2 $OD_{600}$/ml in SC media lacking histidine, uracil, tryptophan, adenine and leucine and supplemented with 10mM 3-aminotriazole, 0.1M sodium phosphate pH 7.0, and 20M fluorescein di-β-D-glucopyranoside (FDGlu). This mixture (50ul per well for 384-well plates, 200ul per well for 96-well plates) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a Spectrofluor microtitre plate reader (Tecan; excitation wavelength: 485nm;

emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[HU210]} - Min_{[HU210]} \times 100\%$$

where $Max_{[compound\ X]}$ and $Min_{[compound\ X]}$ are the fitted maximum and minimum respectively from the concentration effect curve for compound X, and $Max_{[HU210]}$ and $Min_{[HU210]}$ are the fitted maximum and minimum respectively from the concentration effect curve for (6aR,10aR)-3-(1,1'-Dimethylheptyl)-6a,7,10,10a-tetrahydro-1-hydroxy-6,6-dimethyl-6H-dibenzo[b,d]pyran-9-methanol (HU210; available from Tocris). Equieffective molar ratio (EMR) values were calculated from the equation

$$EMR = EC_{50\ [compound\ X]} / EC_{50\ [HU210]}$$

Where $EC_{50\ [compound\ X]}$ is the $EC_{50}$ of compound X and $EC_{50\ [HU210]}$ is the $EC_{50}$ of HU210.
Compounds of Examples 1 to 29 tested according to this method had $EC_{50}$ values 20 to 1000nM and efficacy values of >50% at the cloned human cannabinoid CB2 receptor.
The following examples are illustrative, but not limiting of the embodiments of the present invention.

**All NMR experimental data was recorded at 400MHz.**

**Conditions, Hardware, and Software used for Mass-directed Autopurification**

**Hardware**

Waters 600 gradient pump, Waters 2700 sample manager, Waters Reagent Manager, Micromass ZMD mass spectrometer, Gilson 202 - fraction collector, Gilson Aspec - waste collector

**Software**

Micromass Masslynx version 3.5

**Column**

The column used is typically a Supelco ABZ+ column whose dimensions are 10mm internal diameter by 100mm in length. The stationary phase particle size is 5μm.

**Solvents**

A. Aqueous solvent = Water +0.1% Formic Acid
B. Organic solvent = MeCN: Water 95:5 +0.05% Formic Acid

Make up solvent = MeOH: Water 80:20 +50mMol Ammonium Acetate
Needle rinse solvent = MeOH: Water: DMSO 80:10:10

**Methods**

Five methods are used depending on the analytical retention time of the compound of interest. They all have a flow rate of 20ml/min and a 15-minute runtime, which comprises of a 10-minute gradient followed by a 5-minute column flush and re-equilibration step.
Method 1 MDP 1.5-2.2 = 0-30%B
Method 2 MDP 2.0-2.8 = 5-30% B
Method 3 MDP 2.5-3.0 = 15-55%B
Method 4 MDP 2.8-4.0 = 30-80% B

Method 5 MDP 3.8-5.5 = 50-90% B

**Example 1: 2-(3-Chlorophenylamino)-4-trifluoromethylpyrimidine-5-carboxylic acid benzyl-amide**

(a). To a solution of benzyl 2-chloro-4-trifluoromethylpyrimidine-5-carboxylate (0.50 g, ex Maybridge) in 1,4-dioxan (5 ml) was added 3-chloroaniline (0.85 ml) and the solution stirred at room temperature for 15 h. 1,4-Dioxan was removed under reduced pressure and ethyl acetate (15 ml) added. The solution was washed sequentially with 2N hydrochloric acid (10 ml) and water (3 x 10 ml), dried ($MgSO_4$), evaporated and triturated with hexane to afford benzyl 2-(3-chlorophenylamino)-4-trifluoromethylpyrimidine-5-carboxylate (524 mg).
NMR (400MHz, DMSO-d6) δ 5.35 (2H, s), 7.14 (1H, d), 7.35-7.45 (6H, m), 7.68 (1H, m), 7.98 (1H, s), 9.13 (1H, s), 10.95 (1H, s).
LC/MS, t = 3.70 min, [$MH^+$] 408 and 410.

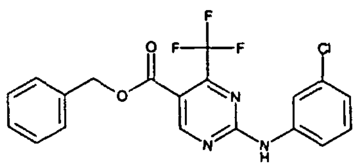

(b). To a solution of benzyl 2-(3-chlorophenylamino)-4-trifluoromethyl-pyrimidine-5-carboxylate (0.50 g) in ethanol (15 ml) was added a solution of potassium hydroxide (205 mg) in ethanol (10 ml) and the solution stirred at reflux for 15 h. Ethanol was removed under reduced pressure and water (15 ml) added. The solution was washed with ether and concentrated hydrochloric acid added to adjust the acidity to pH 1. The precipitated solid was filtered, washed with water and dried in vacuo at 50°C to afford 2-(3-chlorophenylamino)-4-trifluoromethylpyrimidine-5-carboxylic acid (366 mg,).
NMR (400MHz, DMSO-d6) δ 7.49 (1H, d), 7.71 (1H, t), 7.98 (1H, d), 8.33 (1H, s), 9.42 (1H, s), 11.15 (1H,s), 14.0 (1H,brs).
LC/MS, t = 3.44 min, [$MH^+$] 318 and 320.

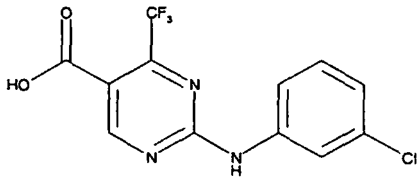

(c). To a solution of 2-(3-chlorophenylamino)-4-trifluoromethylpyrimidine-5-carboxylic acid (35 mg) in dimethylformamide (2 ml) was added successively N-ethylmorpholine (42 μl), benzylamine (15μl), 1-hydroxybenzotriazole hydrate (23 mg) and 1-(3-dimethylamino-propyl)-3-ethylcarbodiimide hydrochloride (25 mg). The solution was stirred for 3 h and allowed to stand overnight. Dimethylformamide was removed under reduced pressure and ethyl acetate (5 ml) added. The solution was washed sequentially with 5% sodium bicarbonate solution (2.5 ml), water (2.5 ml), 5% citric acid solution (2.5 ml) and brine (2 x 2.5 ml), dried ($MgSO_4$) and evaporated to afford the title compound (45 mg).
NMR (400MHz, DMSO-d6) δ 4.47 (2H, d), 7.10 (1H, d), 7.25 (1H, m), 7.36 (5H, m), 7.69 (1H, d), 7.98 (1H, s), 8.89 (1H, s), 9.12 (1H, t), 10.65 (1H, s).
LC/MS, t = 3.23 min, [$MH^+$] 407 and 409.

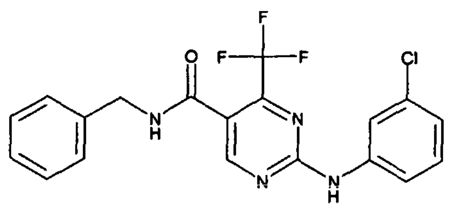

**Example 2: 2-(3-Chlorophenylamino)-4-trifluoromethylpyrimidine-5-carboxylic acid (pyridin-4-ylmethyl)amide**

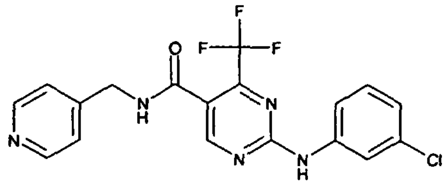

In a manner similar to Example 1(c) 2-(3-chlorophenylamino)-4-trifluoromethylpyrimidine-5-carboxylic acid (35 mg) and 4-(aminomethyl)pyridine (13.5 μl) afforded the title compound (32 mg).
NMR (400MHz, DMSO-d6) δ 4.48 (2H, d), 7.10 (1H, d), 7.37 (3H, m), 7.69 (1H, d), 7.98 (1H, s), 8.55 (2H, d), 8.97 (1H, s), 9.26 (1H, t), 10.65 (1H, s).
LC/MS, t = 2.90 min, [$MH^+$] 408 and 410.

**Example 3: 2-(3-Chlorophenylamino)-4-trifluoromethylpyrimidine-5-carboxylic acid N-benzyl-N-methylamide**

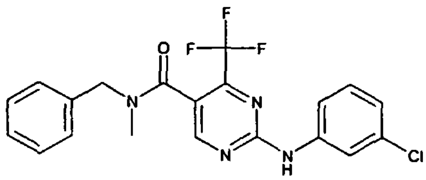

In a manner similar to Example 1(c) 2-(3-chlorophenylamino)-4-trifluoromethylpyrimidine-5-carboxylic acid (35 mg) and N-methylbenzylamine (17 μl) afforded the title compound (46 mg). NMR (400MHz, DMSO-d6) Rotamers in 65: 35 ratio δ 2.88 (1.95H, s), 2.98 (1.05H, s), 4.58 (0.7H, br s), 4.75 (1.3H, br s), 7.17 (1H, t), 7.30 (1H, d), 7.35-7.5 (5H, m), 7.72 (1H, t), 8.00 (0.35H, t), 8.06 (0.65H, t), 8.89 (0.35H, s), 8.95 (0.65H, s), 10.65 (0.35H, s), 10.7 (0.65H, s).
LC/MS, t = 3.35 min, [$MH^+$] 421 and 423.

**Example 4: 2-(3-chlorophenylamino)-4-trifluoromethylpyrimidine-5-carboxylic acid 4-methoxybenzyl-amide**

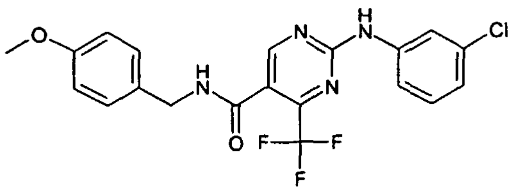

In a manner similar to Example 1(c) 2-(3-chlorophenylamino)-4-trifluoromethylpyrimidine-5-carboxylic acid (35 mg) and 4-methoxybenzylamine (17 μl) afforded the title compound (18 mg).
NMR (400MHz, DMSO-d6) δ 3.75 (3H, s), 4.40 (2H, d), 6.94 (2H, d), 7.10 (1H, d), 7.28 (2H, d), 7.38 (1H, t), 7.69 (1H, d), 7.98 (1H, s), 8.88 (1H, s), 9.08 (1H, t), 10.65 (1H, s).
LC/MS, t = 3.57 min, [$MH^+$] 437 and 439.

**Example 5: 2-(3-Chlorophenylamino)-4-trifluoromethylpyrimidine-5-carboxylic acid 4-fluorobenzyl-amide**

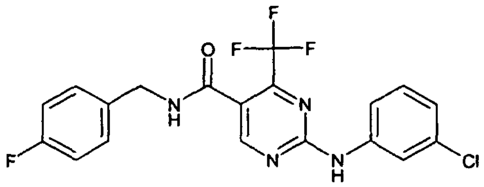

In a manner similar to Example 1(c) 2-(3-chlorophenylamino)-4-trifluoromethylpyrimidine-5-carboxylic acid (35 mg) and 4-fluorobenzylamine hydrochloride (21 mg) afforded the title compound (35 mg).
NMR (400MHz, DMSO-d6) δ 4.45 (2H, d), 7.10 (1H, d), 7.18 (2H, t), 7.35-7.45 (3H, m), 7.68 (1H, d), 7.97 (1H, s), 8.89 (1H, s), 9.14 (1H, t), 10.65 (1H, s).
LC/MS, t = 3.68 min, [$MH^+$] 425 and 427.

**Example 6: 2-(3-Chlorophenylamino)-4-trifluoromethylpyrimidine-5-carboxylic acid 4-cyanobenzyl-amide**

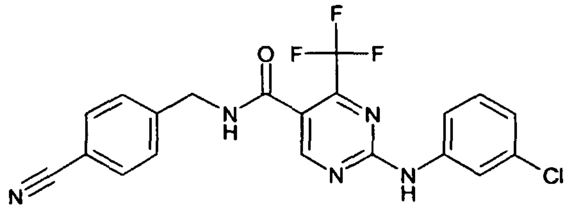

In a manner similar to Example 1(c) 2-(3-chlorophenylamino)-4-trifluoromethylpyrimidine-5-carboxylic acid (35 mg) and 4-cyanobenzylamine (17.5 mg) afforded the title compound (13 mg).
NMR (400MHz, DMSO-d6) δ 4.94 (2H, d), 7.49 (1H, d), 7.68 (1H, t), 7.95 (2H, d), 8.06 (1H, d), 8.23 (2H, d), 8.38 (1H, s), 9.32 (1H, s), 9.64 (1H, t), 11.05 (1H, s).
LC/MS, t = 3.56 min, [$MH^+$] 432 and 434.

**Example 7: 2-(2,3-Dichlorophenylamino)-4-trifluoromethylpyrimidine-5-carboxylic acid N-benzyl-N-methylamide**

(a). To a solution of methyl 2-chloro-4-trifluoromethylpyrimidine-5-carboxylate (0.40 g, ex Maybridge) in 1,4-dioxan (5 ml) was added 2,3-dichloroaniline (1.27 g) and the solution stirred at reflux temperature for 24 h. 1,4-Dioxan was removed under reduced pressure and ethyl acetate (15 ml) added. The solution was washed sequentially with 2N hydrochloric acid (10 ml) and water (3 x 10 ml), dried ($MgSO_4$), evaporated and triturated with hexane to afford methyl 2-(2,3-dichlorophenylamino)-4-trifluoromethyl-pyrimidine-5-carboxylate (176 mg,).
NMR (400MHz, $CDCl_3$) δ 3.97 (3H, s), 7.25 (2H, m), 8.15 (1H, s), 8.48 (1H, d), 9.07 (1H, s). LC/MS, t = 3.68 min, [$MH^+$] 366 and 368.

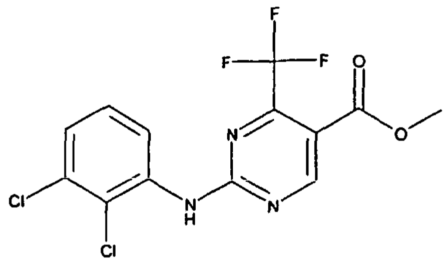

(b). In a manner similar to Example 1(b) methyl 2-(2,3-dichlorophenylamino)-4-trifluoromethylpyrimidine-5-carboxylate (0.18 g) afforded 2-(2,3-dichlorophenylamino)-4-trifluoromethylpyrimidine-5-carboxylic acid (0.13 g).
NMR (400MHz, DMSO-d6) δ 7.40 (1H, t), 7.56 (2H, d), 8.96 (1H, s), 10.45 (1H, s), 13.6 (1H, s).
LC/MS, t = 4.06 min, [$MH^+$-$CO_2$] 306 and 308.

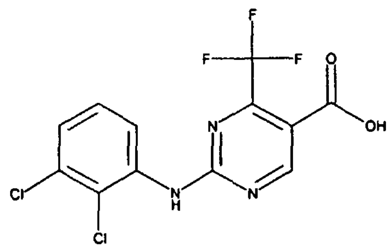

(c). In a manner similar to Example 1(c) 2-(2,3-dichlorophenylamino)-4-trifluoromethylpyrimidine-5-carboxylic acid (39 mg) and N-methylbenzylamine (21.5 μl) afforded the title compound (50 mg).
NMR (400MHz, $CDCl_3$) Rotamers in 65:35 ratio δ 2.79 (1.95H, s), 3.08 (1.05H, s), 4.42 (0.7H, br s), 4.78 (1.3H, br s), 7.14 (1H, d), 7.2-7.3 (2H, m), 7.3-7.45 (4H, m),
7.96 (0.35H, s), 8.01 (0.65H, s), 8.40 (0.35H, d), 8.45 (0.65H, d), 8.55 (0.35H, s), 8.59 (0.65H, s). LC/MS, t = 3.74 min, [$MH^+$] 455 and 457.

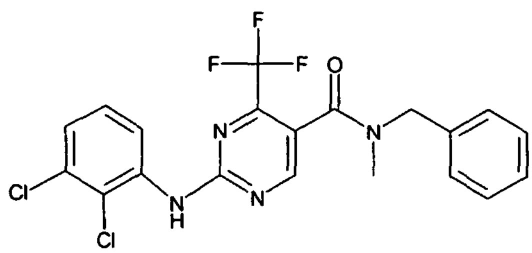

**Example 8: 2-(2,4-Dichlorophenylamino)-4-trifluoromethylpyrimidine-5-carboxylic acid benzyl-amide**

(a). In a manner similar to Example 7(a) methyl 2-chloro-4-trifluoromethylpyrimidine-5-carboxylate (0.5 g) and 2,4-dichloroaniline (1.7 g) afforded methyl 2-(2,4-dichlorophenyl-amino)-4-trifluoromethyl-pyrimidine-5-carboxylate (214 mg).
NMR (400MHz, $CDCl_3$) δ 3.95 (3H, s), 7.33 (1H, d), 7.46 (1H, d), 7.99 (1H, s), 8.48 (1H, d), 9.06 (1H, s).
LC/MS, t = 3.74 min, [$MH^+$] 366 and 368.

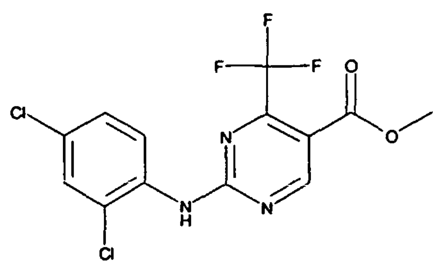

(b). In a manner similar to Example 1(b) methyl 2-(2,4-dichlorophenylamino)-4-trifluoromethylpyrimidine-5-carboxylate (0.21 g) afforded 2-(2,4-dichlorophenylamino)-4-trifluoromethylpyrimidine-5-carboxylic acid (0.18 g).
NMR (400MHz, DMSO-d6) δ 7.47 (1H, d), 7.60 (1H, d), 7.75 (1H, s), 8.96 (1H, s), 10.3 (1H, s), 13.6 (1H, s).
LC/MS, t = 4.17 min, [$MH^+$-$CO_2$] 306 and 308.

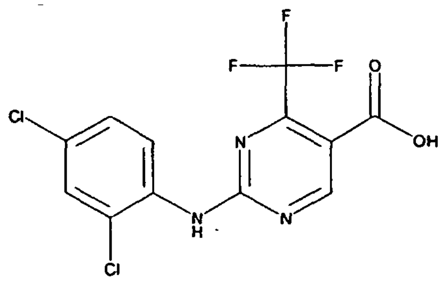

(c). In a manner similar to Example 1(c) 2-(2,4-dichlorophenylamino)-4-trifluoromethylpyrimidine-5-carboxylic acid (39 mg) and benzylamine (18 μl) afforded the title compound (41 mg).
NMR (400MHz, $CDCl_3$) δ 4.64 (2H, d), 6.08 (1H, br s), 7.25-7.4 (5H, m), 7.44 (1H, d), 7.90 (1H, s), 8.43 (1H, d), 8.74 (1H, s).
LC/MS, t = 3.69 min, [$MH^+$] 441 and 443.

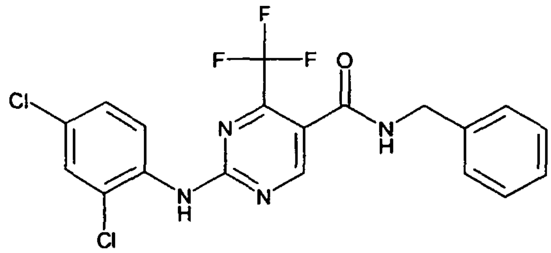

**Example 9: 2-(3,4-Dichlorophenylamino)-4-trifluoromethylpyrimidine-5-carboxylic acid benzyl-amide**

(a). In a manner similar to Example 7(a) methyl 2-chloro-4-trifluoromethylpyrimidine-5-carboxylate (0.5 g) and 3,4-dichloroaniline (1.7 g) afforded methyl 2-(3,4-dichlorophenyl-amino)-4-trifluoromethyl-pyrimidine-5-carboxylate (591 mg).
NMR (400MHz, $CDCl_3$) δ 3.96 (3H, s), 7.45 (2H, m), 7.57 (1H, s), 7.98 (1H, s), 9.07 (1H, s). LC/MS, t = 3.87 min, [$MH^+$] 366 and 368.

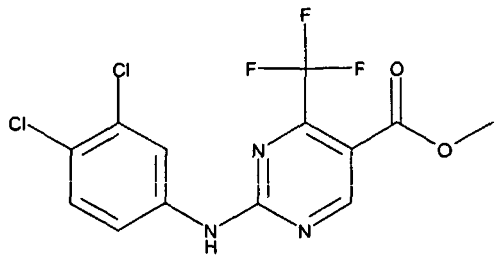

(b). In a manner similar to Example 1(b) methyl 2-(3,4-dichlorophenylamino)-4-trifluoromethylpyrimidine-5-carboxylate (0.59 g) afforded 2-(3,4-dichlorophenylamino)-4-trifluoromethylpyrimidine-5-carboxylic acid (0.51 g).
NMR (400MHz, DMSO-d6) δ 7.65 (1H, d), 7.72 (1H, d of d), 8.19 (1H, s), 9.12 (1H, s), 10.95 (1H, s), 13.7 (1H, s).
LC/MS, t = 4.49 min, [$MH^+$-$CO_2$] 306 and 308.

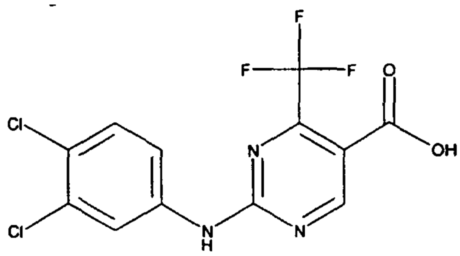

(c). In a manner similar to Example 1(c) 2-(3,4-dichlorophenylamino)-4-trifluoromethylpyrimidine-5-carboxylic acid (39 mg) and benzylamine (18 μl) afforded the title compound (51 mg).
NMR (400MHz, $CDCl_3$) δ 4.65 (2H, d), 6.10 (1H, br s), 7.3-7.4 (5H, m), 7.42 (1H, s), 7.45 (1H, s), 7.94 (1H, s), 8.78 (1H, s).
LC/MS, t = 3.80 min, [$MH^+$] 441 and 443.

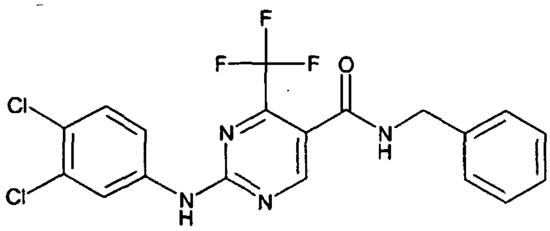

**Example 10: 2-(2,6-Dichlorophenylamino)-4-trifluoromethylpyrimidine-5-carboxylic acid N-benzyl-N-methylamide**

(a). In a manner similar to Example 7(a) methyl 2-chloro-4-trifluoromethylpyrimidine-5-carboxylate (0.5 g) and 2,6-dichloroaniline (1.7 g) in 1,4-dioxan (5 ml) was stirred at reflux temperature for 7 days to afford methyl 2-(2,6-dichlorophenyl-amino)-4-trifluoromethylpyrimidine-5-carboxylate (136 mg).
LC/MS, t = 3.43 min, [$MH^+$] 366 and 368.

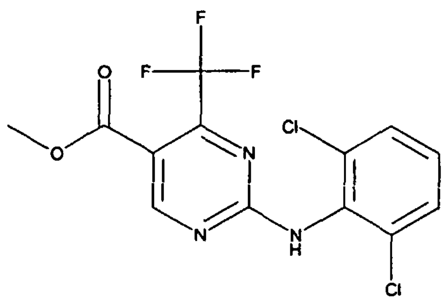

(b). In a manner similar to Example 1(b) methyl 2-(2,6-dichlorophenylamino)-4-trifluoromethylpyrimidine-5-carboxylate (135 mg) afforded 2-(2,6-dichlorophenylamino)-4-trifluoromethylpyrimidine-5-carboxylic acid (114 mg).
NMR (400MHz, DMSO-d6) δ 7.41 (1H, t), 7.60 (2H, d), 8.92 (1H, br s), 10.5 (1H, s), 13.6 (1H, br s).

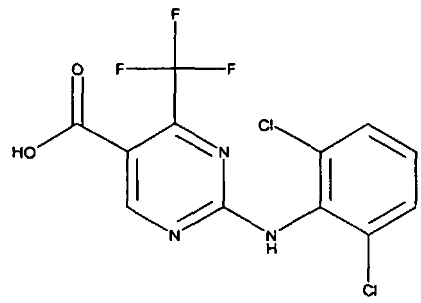

(c). In a manner similar to Example 1(c) 2-(2,6-dichlorophenylamino)-4-trifluoromethylpyrimidine-5-carboxylic acid (40 mg) and N-methylbenzylamine (18 μl) afforded the title compound (49 mg).
NMR (400MHz, DMSO-d6) Rotamers in 65:35 ratio δ 2.83 (1.95H, s), 2.98 (1.05H, s), 4.51 (0.7H, s), 4.74 (1.3H, br s), 7.26 (1H, d), 7.3-7.5 (5H, m), 7.65 (2H, t), 8.69 (0.35H, br s), 8.78 (0.65H, br s), 10.3 (1H, s).
LC/MS, t = 3.51 min, [$MH^+$] 455 and 457.

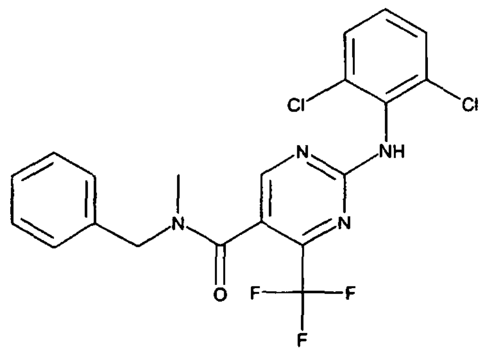

**Example 11: 2-(3,5-Dichlorophenylamino)-4-trifluoromethylpyrimidine-5-carboxylic acid benzyl-amide**

(a). In a manner similar to Example 7(a) methyl 2-chloro-4-trifluoromethylpyrimidine-5-carboxylate (0.5 g) and 3,5-dichloroaniline (1.7 g) afforded methyl 2-(3,5-dichlorophenyl-amino)-4-trifluoromethyl-pyrimidine-5-carboxylate (0.76 g).
LC/MS, t = 3.96 min, [$MH^+$] 366 and 368.

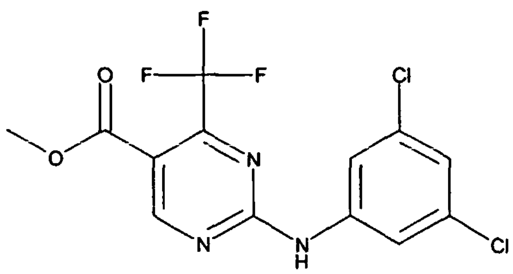

(b). In a manner similar to Example 1(b) methyl 2-(3,5-dichlorophenylamino)-4-trifluoromethylpyrimidine-5-carboxylate (0.76g) afforded 2-(3,5-dichlorophenylamino)-4-trifluoromethylpyrimidine-5-carboxylic acid (0.65 g).
NMR (400MHz, DMSO-d6) δ 7.28 (1H, s), 7.90 (2H, s), 9.14 (1H, s), 10.95 (1H, s), 13.75 (1H, br s).

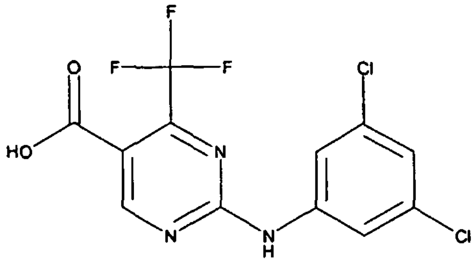

(c). In a manner similar to Example 1(c) 2-(3,5-dichlorophenylamino)-4-trifluoromethylpyrimidine-5-carboxylic acid (35 mg) and benzylamine (13 μl) afforded the title compound (29 mg).
NMR (400MHz, DMSO-d6) δ 4.48 (2H, d), 7.25 (2H, m), 7.38 (4H, m), 7.89 (2H, s), 8.95 (1H, s), 9.16 (1H, t), 10.8 (1H, s).
LC/MS, t = 3.87 min, [$MH^+$] 441 and 443.

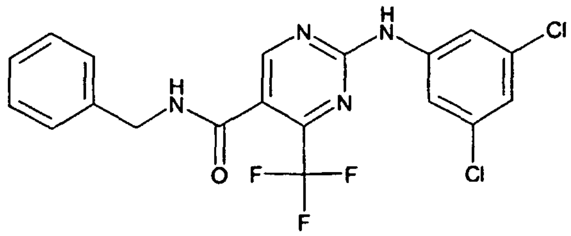

**Example 12: 2-(3-Fluorophenylamino)-4-trifluoromethylpyrimidine-5-carboxylic acid benzyl-amide**

(a). In a manner similar to Example 1(a) methyl 2-chloro-4-trifluoromethylpyrimidine-5-carboxylate (0.5 g) and 3-fluoroaniline (1.16 g) afforded methyl 2-(3-fluorophenylamino)-4-trifluoromethyl-pyrimidine-5-carboxylate (0.65 g).
NMR (400MHz, DMSO-d6) δ 3.88 (3H, s), 6.95 (1H, t of d), 7.40 (1H, q), 7.54 (1H, d), 7.79 (1H, d of t), 9.12 (1H, s), 10.95 (1H, s).
LC/MS, t = 3.50 min, [$MH^+$] 316.

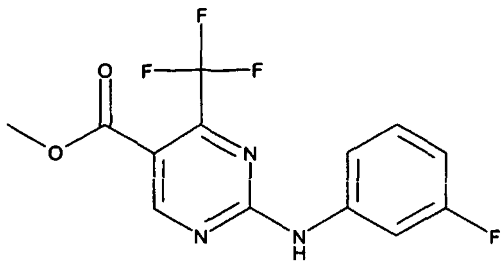

(b). In a manner similar to Example 1(b) methyl 2-(3-fluorophenylamino)-4-trifluoromethylpyrimidine-5-carboxylate (0.65g) afforded 2-(3-fluorophenylamino)-4-trifluoromethylpyrimidine-5-carboxylic acid (0.54 g).
NMR (400MHz, DMSO-d6) δ 6.90 (1H, t of d), 7.39 (1H, q), 7.55 (1H, d), 7.80 (1H, d of t), 9.10 (1H, s), 10.85 (1H, s), 13.7 (1H, br s).

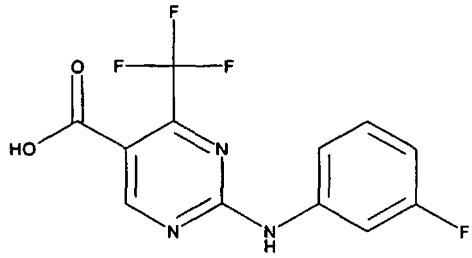

(c). In a manner similar to Example 1(c) 2-(3-fluororophenylamino)-4-trifluoromethylpyrimidine-5-carboxylic acid (35 mg) and benzylamine (15 μl) afforded the title compound (35 mg).
NMR (400MHz, DMSO-d6) δ 4.46 (2H, d), 6.87 (1H, t of d), 7.28 (1H, m), 7.35 (5H, m), 7.52 (1H, d), 7.78 (1H, d of t), 8.89 (1H, s), 9.15 (1H, t), 10.65 (1H, s).
LC/MS, t = 3.47 min, [$MH^+$] 391.

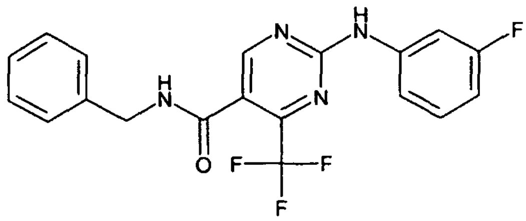

**Example 13: 2-(3-Bromophenylamino)-4-trifluoromethylpyrimidine-5-carboxylic acid benzyl-amide**

(a). In a manner similar to Example 1(a) methyl 2-chloro-4-trifluoromethylpyrimidine-5-carboxylate (0.5 g) and 3-bromoaniline (1.79 g) afforded methyl 2-(3-bromophenylamino)-4-trifluoromethyl-pyrimidine-5-carboxylate (0.68 g).
NMR (400MHz, DMSO-d6) δ 3.88 (3H, s), 7.30 (2H, m), 7.72 (1H, d), 8.12 (1H, s), 9.11 (1H, s), 10.90 (1H, s).
LC/MS, t = 3.70 min, [$MH^+$] 376 and 378.

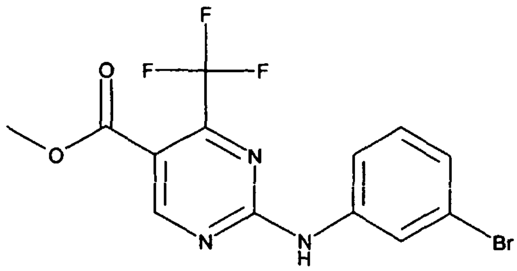

(b). In a manner similar to Example 1(b) methyl 2-(3-bromophenylamino)-4-trifluoromethylpyrimidine-5-carboxylate (0.68 g) afforded 2-(3-bromophenylamino)-4-trifluoromethylpyrimidine-5-carboxylic acid (0.57 g).
NMR (400MHz, DMSO-d6) δ 7.30 (2H, m), 7.73 (1H, d), 8.15 (1H, s), 9.09 (1H, s), 10.80 (1H, s), 13.65 (1H, br s).

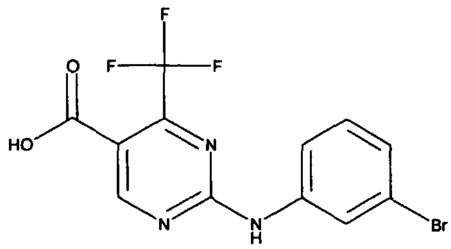

(c). In a manner similar to Example 1(c) 2-(3-bromophenylamino)-4-trifluoromethyl-pyrimidine-5-carboxylic acid (35 mg) and benzylamine (13 μl) afforded the title compound (23 mg).
NMR (400MHz, DMSO-d6) δ 4.47 (2H, d), 7.2-7.4 (7H, m), 7.71 (1H, d), 8.11 (1H, s), 8.89 (1H, s), 9.15 (1H, t), 10.65 (1H,s).
LC/MS, t = 3.64 min, [$MH^+$] 451 and 453.

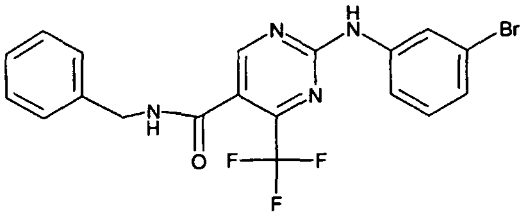

**Example 14:2-(3-Bromophenylamino)-4-trifluoromethylpyrimidine-5-carboxylic acid N-benzyl-N-methylamide**

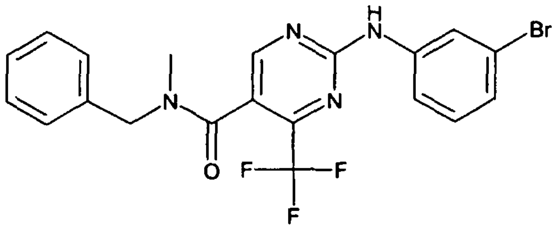

In a manner similar to Example 1(c) 2-(3-bromophenylamino)-4-trifluoromethyl-pyrimidine-5-carboxylic acid (35 mg) and N-methylbenzylamine (15 μl) afforded the title compound (45 mg). NMR (400MHz, DMSO-d6) Rotamers in 65:35 ratio δ 2.89 (1.95H, s), 2.98 (1.05H, s), 4.58 (0.7H, br s), 4.76 (1.3H, br s), 7.28 (1H, d), 7.25-7.5 (6H, m), 7.76 (1H, t), 8.13 (0.35H, t), 8.19 (0.65H, t), 8.88 (0.35H, s), 8.95 (0.65H, s), 10.6 (0.35H, s), 10.65 (0.65H, s).
LC/MS, t = 3.72 min, [$MH^+$] 465 and 467.

**Example 15: 2-(2-Methoxyphenylamino)-4-trifluoromethylpyrimidine-5-carboxylic acid benzyl-amide**

(a). In a manner similar to Example 1(a) benzyl 2-chloro-4-trifluoromethylpyrimidine-5-carboxylate (0.5 g) and 2-methoxyaniline (0.97 g) afforded benzyl 2-(2-methoxyphenyl-amino)-4-trifluoromethyl-pyrimidine-5-carboxylate (0.57 g).
NMR (400MHz, $CDCl_3$) δ 3.93 (3H, s), 5.38 (2H, s), 6.93 (1H, d), 7.04 (1H, t), 7.09 (1H, t), 7.35-7.45 (4H, m), 8.26 (1H, br s), 8.49 (1H, br d), 9.06 (1H, s).
LC/MS, t = 3.42 min, [$MH^+$] 404.

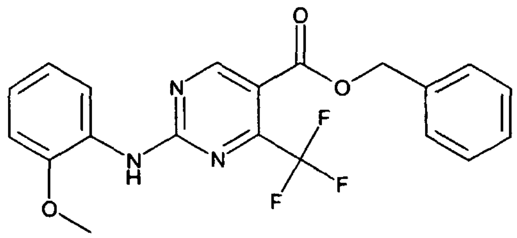

(b). In a manner similar to Example 1(b) benzyl 2-(2-methoxyphenylamino)-4-trifluoromethylpyrimidine-5-carboxylate (0.55 g) afforded 2-(2-methoxyphenylamino)-4-trifluoromethylpyrimidine-5-carboxylic acid (0.38 g).
NMR (400MHz, DMSO-d6) δ 3.80 (3H, s), 6.98 (1H, t), 7.10 (1H, d), 7.22 (1H, t), 7.62 (1H, d), 8.94 (1H, s), 9.62 (1H, s), 13.5 (1H, s).
LC/MS, t = 3.03 min, [$MH^+$] 314.

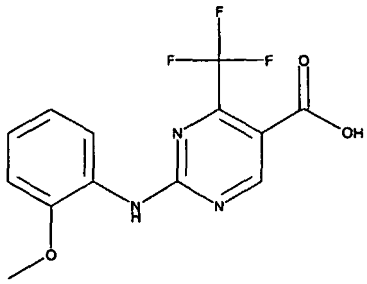

(c). In a manner similar to Example 1(c) 2-(2-methoxyphenylamino)-4-trifluoromethylpyrimidine-5-carboxylic acid (35 mg) and benzylamine (18 μl) afforded, after silica gel chromatography using 1:1 ethyl acetate:isohexane, the title compound (38 mg).
NMR (400MHz, $CDCl_3$) δ 3.93 (3H, s), 4.65 (2H, d), 6.09 (1H, br s), 6.90 (1H, d), 7.05 (2H, m), 7.35 (5H, m), 8.25 (1H, s), 8.47 (1H, d), 8.75 (1H, s).
LC/MS, t = 3.14 min, [$MH^+$] 403.

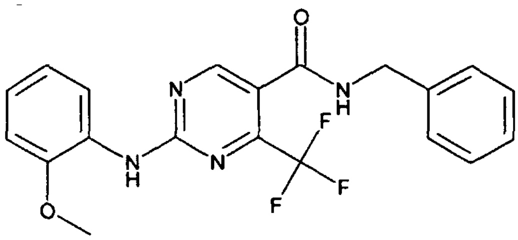

**Example 16: 2-(2,3-Dichlorophenylamino)-4-trifluoromethylpyrimidine-5-carboxylic acid pyridin-4-ylmethyl) amide**

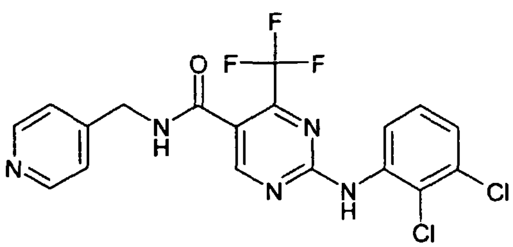

In a manner similar to Example 1(c) 2-(2,3-dichlorophenylamino)-4-trifluoromethyl-pyrimidine-5-carboxylic acid

(24 mg) and 4-(aminomethyl)pyridine (10 μl) afforded the title compound (19 mg).
NMR (400MHz, DMSO-d6)δ 4.48 (2H, d), 7.34 (2H, d), 7.41 (1H, t), 7.55 (2H, m), 8.52 (2H, d), 8.81 (1H, s), 9.23 (1H, t), 10.20 (1H, s).
LC/MS, t = 2.95 min, [MH+] 442 and 444.

**Example 17: 2-(2,4-Dichlorophenylamino)-4-trifluoromethylpyrimidine-5-carboxylic acid (pyridin-4-ylmethyl) amide**

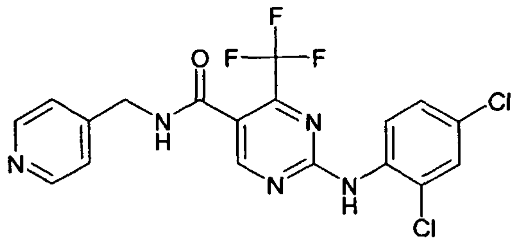

In a manner similar to Example 1(c) 2-(2,4-dichlorophenylamino)-4-trifluoromethyl-pyrimidine-5-carboxylic acid (30 mg) and 4-(aminomethyl)pyridine (10.5 μl) afforded the title compound (24 mg).
NMR (400MHz, DMSO-d6) δ 4.48 (2H, d), 7.33 (2H, d), 7.48 (1H, d of d), 7.60 (1H, d), 7.75 (1H, s), 8.52 (2H, d), 8.80 (1H, s), 9.22 (1H, t), 10.10 (1H, s).
LC/MS, t = 3.00 min, [MH+] 442 and 444.

**Example 18: 2-(3,4-Dichlorophenylamino)-4-trifluoromethylpyrimidine-5-carboxylic acid (pyridin-4-ylmethyl) amide**

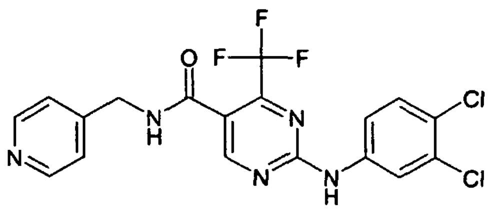

In a manner similar to Example 1(c) 2-(3,4-dichlorophenylamino)-4-trifluoromethyl-pyrimidine-5-carboxylic acid (30 mg) and 4-(aminomethyl)pyridine (10.5 μl) afforded the title compound (32 mg).
NMR (400MHz, DMSO-d6) δ 4.50 (2H, d), 7.36 (2H, d), 7.62 (1H, d), 7.70 (1H, d of d), 8.18 (1H, s), 8.55 (2H, d), 8.99 (1H, s), 9.27 (1H, t), 10.75 (1H, s).
LC/MS , t = 3.17 min, [MH+] 442 and 444.

**Example 19: 2-(2,5-Dichlorophenylamino)-4-trifluoromethylpyrimidine-5-carboxylic acid (pyridin-4-ylmethyl) amide**

(a). In a manner similar to Example 8(a) methyl 2-chloro-4-trifluoromethylpyrimidine-5-carboxylate (0.5 g) and 2,5-dichloroaniline (1.7 g) afforded methyl 2-(2,5-dichlorophenyl-amino)-4-trifluoromethylpyrimidine-5-carboxylate (681 mg).
LC/MS, t = 3.73 min, [MH+] 366 and 368.

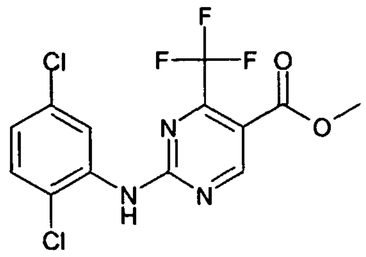

(b). In a manner similar to Example 1(b) methyl 2-(2,5-dichlorophenylamino)-4-trifluoromethylpyrimidine-5-carboxylate (0.68 g) afforded 2-(2,5-dichlorophenylamino)-4-trifluoromethylpyrimidine-5-carboxylic acid (0.48 g).
NMR (400MHz, DMSO-d6) δ 7.36 (1H, d of d), 7.60 (1H, d), 7.76 (1H, d), 8.99 (1H, s), 10.3 (1H, s), 13.6 (1H, br s).

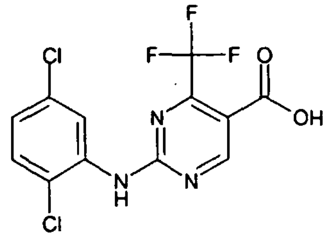

In a manner similar to Example 1(c) 2-(2,5-dichlorophenylamino)-4-trifluoromethyl-pyrimidine-5-carboxylic acid (35 mg) and 4-(aminomethyl)pyridine (17 μl) afforded the title compound (35 mg).
NMR (400MHz, DMSO-d6) δ 4.47 (2H, d), 7.33 (3H, m), 7.60 (1H, d), 7.73 (1H, d), 8.54 (2H, d), 8.84 (1H, s), 9.21 (1H, t), 10.10 (1H, s).
LC/MS, t = 2.96 min, [$MH^+$] 442 and 444.

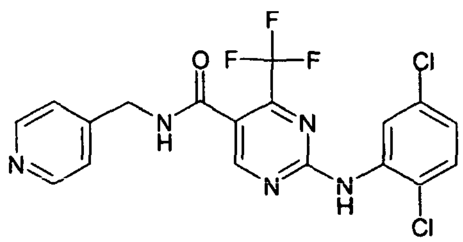

**Example 20: 2-(3-Fluorophenylamino)-4-trifluoromethylpyrimidine-5-carboxylic acid (pyridin-4-ylmethyl) amide**

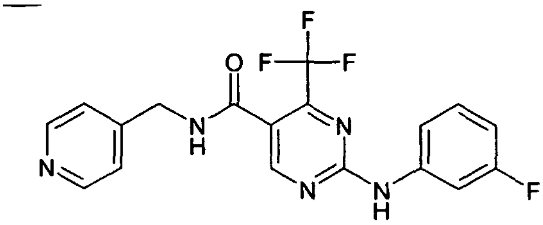

In a manner similar to Example 1(c) 2-(3-fluorophenylamino)-4-trifluoromethylpyrimidine-5-carboxylic acid (35 mg) and 4-(aminomethyl)pyridine (12 μl) afforded the title compound (28 mg).
NMR (400MHz, DMSO-d6) δ 4.51 (2H, d), 6.88 (1H, t of d), 7.4 (3H, m), 7.55 (1H, d), 7.80 (1H, d of t), 8.56 (2H, d), 8.96

(1H, s), 9.26 (1H, t), 10.70 (1H, s).
LC/MS, t = 2.65 min, [MH+] 392.

**Example 21: 2-(3-Bromophenylamino)-4-trifluoromethylpyrimidine-5-carboxylic acid (pyridin-4-ylmethyl) amide**

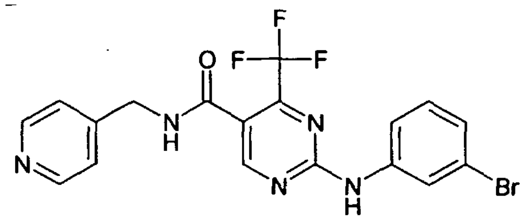

In a manner similar to Example 1(c) 2-(3-bromophenylamino)-4-trifluoromethylpyrimidine-5-carboxylic acid (35 mg) and 4-(aminomethyl)pyridine (12 μl) afforded the title compound (29 mg).
NMR (400MHz, DMSO-d6) δ 4.48 (2H, d), 7.24 (1H, d), 7.34 (1H, t), 7.38 (2H, d), 7.73 (1H, d), 8.13 (1H, s), 8.56 (2H, d), 8.98 (1H, s), 9.26 (1H, t), 10.65 (1H, s).
LC/MS, t = 2.91 min, [MH+] 452 and 454.

**Example 22: 2-(3,5-Dichlorophenylamino)-4-trifluoromethylpyrimidine-5-carboxylic acid (pyridin-4-ylmethyl) amide**

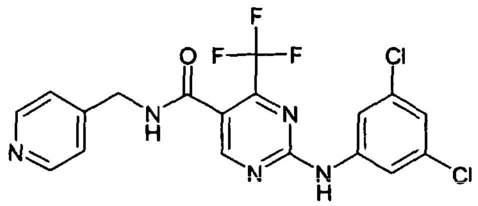

In a manner similar to Example 1(c) 2-(3,5-dichlorophenylamino)-4-trifluoromethyl-pyrimidine-5-carboxylic acid (35 mg) and 4-(aminomethyl)pyridine (17 μl) afforded the title compound (39 mg).
NMR (400MHz, DMSO-d6) δ 4.48 (2H, d), 7.26 (1H, s), 7.36 (2H, d), 7.89 (2H, s), 8.55 (2H, d), 9.03 (1H, s), 9.29 (1H, t), 10.85 (1H, s).
LC/MS, t = 2.96 min, [MH+] 442 and 444.

**Example 23: 2-(3-Fluorophenylamino)-4-trifluoromethylpyrimidine-5-carboxylic acid 4-fluorobenzyl-amide**

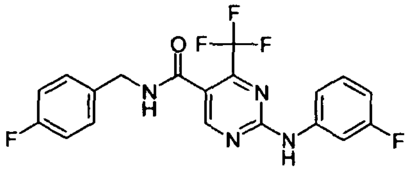

In a manner similar to Example 1(c) 2-(3-fluorophenylamino)-4-trifluoromethyl-pyrimidine-5-carboxylic acid (35 mg) and 4-fluorobenzylamine hydrochloride (22.5 mg) afforded the title compound (31 mg).
NMR (400MHz, DMSO-d6) δ 4.46 (2H, d), 6.88 (1H, t), 7.19 (2H, t), 7.35-7.45 (3H, m), 7.53 (1H, d), 7.78 (1H, d oft),

8.89 (1H, s), 9.15 (1H, t), 10.65 (1H, s).
LC/MS, t = 3.49 min, [MH+] 409.

**Example 24:2-(3-Bromophenylamino)-4-trifluoromethylpyrimidine-5-carboxylic acid 4-fluorobenzyl-amide**

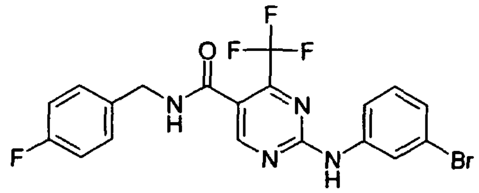

In a manner similar to Example 1(c) 2-(3-bromophenylamino)-4-trifluoromethyl-pyrimidine-S-carboxylic acid (35 mg) and 4-fluorobenzylamine hydrochloride (19 mg) afforded the title compound (31 mg).
NMR (400MHz, DMSO-d6) δ 4.44 (2H, d), 7.15-7.25 (3H, m), 7.31 (1H, t), 7.4 (2H, m), 7.71 (1H, d), 8.10 (1H, s), 8.88 (1H, s), 9.14 (1H, t), 10.60 (1H, s).
LC/MS, t = 3.65 min, [MH+] 469 and 471.

**Example 25: 2-(3,5-Dichlorophenylamino)-4-trifluoromethylpyrimidine-5-carboxylic acid 4-fluorobenzyl-amide**

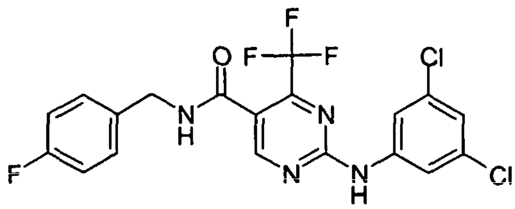

In a manner similar to Example 1(c) 2-(3,5-dichlorophenylamino)-4-trifluoromethyl-pyrimidine-5-carboxylic acid (33 mg) and 4-fluorobenzylamine hydrochloride (17 mg) afforded the title compound (33 mg).
NMR (400MHz, DMSO-d6) δ 4.44 (2H, d), 7.19 (2H, t), 7.26 (1H, s), 7.40 (2H, t), 7.88 (2H, s), 8.94 (1H, s), 9.16 (1H, t), 10.80 (1H, s).
LC/MS, t = 3.87 min, [MH+] 459 and 457.

**Example 26: 2-(3,5-Dichlorophenylamino)-4-trifluoromethylpyrimidine-5-carboxylic acid 4-cyanobenzylamide**

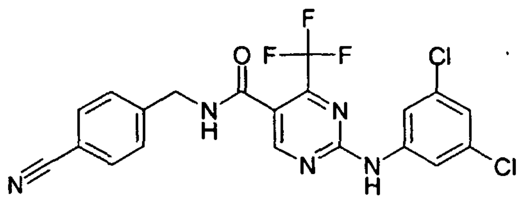

In a manner similar to Example 1(c) 2-(3,5-dichlorophenylamino)-4-trifluoromethyl-pyrimidine-5-carboxylic acid (35 mg) and 4-cyanobenzylamine (20 mg) afforded the title compound (33 mg). NMR (400MHz, DMSO-d6)δ 4.55 (2H, d), 7.26 (1H, s), 7.56 (2H, d), 7.84 (2H, d), 7.88 (2H, s), 8.99 (1H, s), 9.27 (1H, t), 10.80 (1H, s).
LC/MS, t = 3.74 min, [MH+] 466 and 468.

**Example 27: 2-(4-Cyano-phenylamino)-4-trifluoromethyl-pyrimidine-5-carboxylic acid benzylamide**

(a). In a manner similar to Example 1(a) benzyl 2-chloro-4-trifluoromethylpyrimidine-5-carboxylate (0.5 g) and 4-aminobenzonitrile (0.93 g) afforded, after silica gel chromatography using 3:2 isohexane:ethyl acetate, benzyl 2-(4-cyanophenyl-amino)-4-trifluoromethylpyrimidine-5-carboxylate (323 mg).
NMR (400MHz, $CDCl_3$)δ 5.39 (2H, s), 7.35-7.5 (5H, m), 7.68 (2H, d), 7.76 (1H, s), 7.84 (2H, d), 9.10 (1H, s).
LC/MS CF100603-1, t = 3.39 min, [$MH^+$] 399.

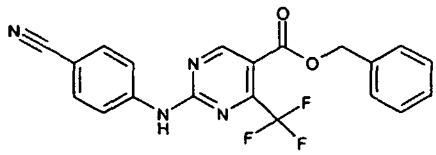

(b). To a solution of benzyl 2-(4-cyanophenylamino)-4-trifluoromethyl-pyrimidine-5-carboxylate (323 mg) in dimethylformamide (6 ml) was added 10% palladium on charcoal (wet) and the mixture stirred under atmospheric hydrogenation conditions for 4 h. Catalyst was filtered through a 1 μM PTFE filter and filtrate evaporated under reduced pressure. The residual solid was triturated with ether, filtered and dried *in vacuo* at 50°C to afford 2-(4-cyanophenylamino)-4-trifluoromethylpyrimidine-5-carboxylic acid (284 mg).
NMR (400MHz, DMSO-d6) δ 7.84 (2H, d), 7.99 (2H, d), 9.13 (1H, s), 11.1 (1H, s), 13.8 (1H, br s). LC/MS CF100887-1, t = 2.78 min, [$MH^+$] 309.

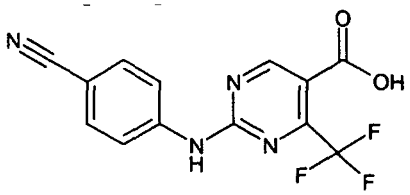

(c) In a manner similar to example 1(c), 2-(4-cyanophenylamino)-4-trifluoromethylpyrimidine-5-carboxylic acid and benzylamine afforded the title compound LC/MS, t = 3.02 min, [$MH^+$] 398.

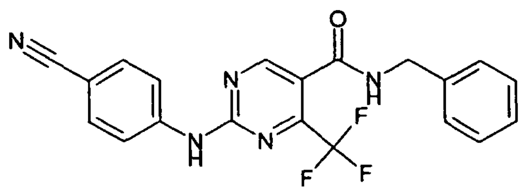

**Example 28: 2-(3-Chloro-phenylamino)-4-trifluoromethyl-pyrimidine-5-carboxylic acid (2-methyl-pyridin-4-ylmethyl)-amide hydrochloride**

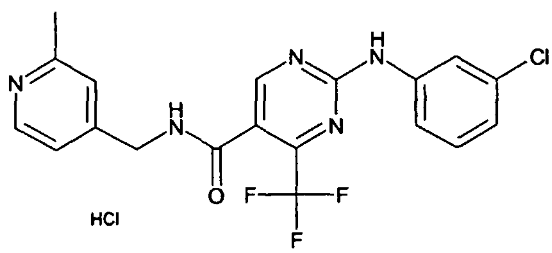

To a solution of 2-(3-chloro-phenylamino)-4-trifluoromethyl-pyrimidine-5-carboxylic acid (2-methyl-pyridin-4-ylmethyl)-amide (15 mg) in ethanol (2 ml) was added a few drops of concentrated hydrochloric acid. The solution was stirred at room temperature for 0.5 h and then evaporated under reduced pressure. Trituration with ether precipitated a white solid which was filtered off, washed with fresh ether and dried to afford the title compound (14 mg).
NMR (400MHz, DMSO-d6) δ 2.72 (3H, s), 4.67 (2H, d), 7.12 (1H, d), 7.38 (1H, t), 7.67 (1H, d), 7.75 (1H, d), 7.79 (1H, s), 8.00 (1H, s), 8.71 (1H, d), 9.06 (1H, s), 9.49 (1H, t), 10.70 (1H, s) LC/MS, t = 2.62 min, [$MH^+$] 422 and 424.

**Example 29: 2-(3-Chloro-phenylamino)-4-trifluoromethyl-pyrimidine-5-carboxylic acid (2-fluoro-pyridin-4-ylmethyl)-amide.**

(a). 4-Bromomethyl-2-fluoro-pyridine.

To a solution of 2-fluoro-4-methylpyridine (1.0 g, ex Lancaster) in carbon tetrachloride (10 ml) was added N-bromosuccinimide (1.6 g, ex Lancaster) and 1,1'- azobis (cyclohexanecarbonitrile) (100 mg, ex Aldrich). The mixture was then refluxed for 24h. Carbon tetrachloride was removed under reduced pressure and the crude oily solid was used in the next stage without purification. LC/MS, t = 2.38 min, [$MH^+$] 190 and 192.

(b). (2-Fluoro-pyridin-4-ylmethyl)-carbamic acid tert-butyl ester.

To crude 4-bromomethyl-2-fluoro-pyridine in an ice bath was added 25% ammonia solution (10 ml, ex BDH) and the mixture stirred at 0° for 5h. Ammonia solution was removed under reduced pressure and the yellow oily solid residue dissolved in dichloromethane (10 ml) and dimethylformamide (1 ml). The solution was cooled in an ice bath and triethylamine (1.5 ml, ex BDH) was added followed by di-tert-butyl dicarbonate (1.0 g, ex Avocado). The solution was stirred at 0°for 1h and then the dichloromethane removed under reduced pressure. The residue was dissolved in ethyl acetate and washed twice with water, dried ($MgSO_4$) and evaporated to give a yellow oil. This was purified by Biotage chromatography (100 g, silica column) eluting with 30% ethyl acetate in hexane to afford the title compound as a white solid (358 mg).
NMR (400MHz, DMSO-d6) δ 1.40 (9H, s), 4.20 (2H, d), 6.97 (1H, s), 7.20 (1H, d), 7.60 (1H, t), 8.17 (1H, d)
LC/MS, t = 2.60 min, [M - Me2C=CH2 + H]$^+$ 171

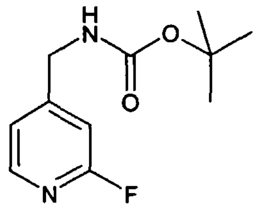

(c). C-(2-Fluoro-pyridin-4-yl)-methylamine dihydrochloride.

(2-Fluoro-pyridin-4-ylmethyl)-carbamic acid tert-butyl ester (350mg) was treated at room temperature with 4N hydrochloric acid in 1,4-dioxan (5 ml) and stirred for 2h. The white precipitate was filtered, washed with fresh ether and dried to afford the title compound (200 mg). NMR (400MHz, DMSO-d6) δ 4.14 (2H, d), 7.38 (1H, s), 7.51 (1H, d), 8.28 (1H, d), 8.82 (3H, s).

(d). 2-(3-Chloro-phenylamino)-4-trifluoromethyl-pyrimidine-5-carboxylic acid (2-fluoro-pyridin-4-ylmethyl)-amide

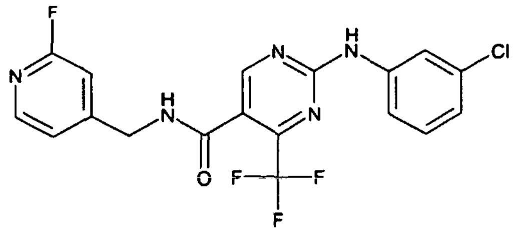

In a manner similar to Example 1(c) 2-(3-chloro-phenylamino)-4-trifluoromethyl-pyrimidine-5-carboxylic acid (75 mg) and C-(2-fluoro-pyridin-4-yl)-methylamine dihydrochloride (56 mg) afforded the title compound (85 mg).
NMR (400MHz, DMSO-d6) δ 4.55 (2H, d), 7.10 (2H, m), 7.38 (2H, m), 7.66 (1H, m), 7.98 (1H, m), 8.21 (1H, d), 8.99 (1H, s), 9.29 (1H, t), 10.65 (1H, s)
LC/MS, t = 3.33 min, $[MH^+]$ 426 and 428.

Example 115: Preparation of Nanomilled Compound

2.5 g of a compound is weighed into a 10 ml centrifuge tube. 10 ml of 0.3mm YTZ milling beads (Manufacturer, batch no.) are weighed into a 50 ml milling pot. 22.5 ml of aqueous 1.5% HPMC is measured with a measuring cylinder into a 100 ml beaker. This solution is homogenised for 3 seconds with an Ultra Turrax T25 homogeniser. Approximately 200 mg of the 2.5 g of the compound is added to the HPMC solution and homogenised at the lowest speed setting until the powder is wetted. This is repeated until all the compound is added. The speed of the homogeniser is then increased to maximum and the suspension is homogenised for a further 3 minutes. This suspension is allowed to stand for 30 minutes in order to allow some of the foam to disperse. The suspension is then poured into the 50 ml pot containing the YTZ milling beads, stirring to release any trapped air. The lid to the pot is then fitted and the pot sealed with some Nesco film. This procedure is repeated for a second 50 ml nanomilling pot and both pots are placed on a Retsch mill and milled for a total of 8 hours.

The milling pots are removed from the Retsch mill and left to cool and for the foam to disperse overnight. In the morning the suspension and bead mixture is passed through a 200μ, 40 mm diameter screen. The contents from each 50 ml pot is washed with aqueous 1.5% HPMC: 10% of the original suspension volume (i.e. 2.5 ml). The suspension from the 2 pots is combined to make 1 batch. The suspension obtained from the method above is named the concentrate.

The concentrate is diluted 1 in 4 with aqueous 1.5% HPMC to give a nominal concentration of 25 mg/ml. This first dilution is assayed by HPLC to calculate the actual concentration

**HPLC conditions**

Column: Symmetry $C_{18}$ 5μ 3.9x150 mm column; flow rate 1.0 ml/min; column temp 40°C.; UV detection at 280nm.

Mobile phase gradient: A: water + 0.1 % trifluoro acetic acid (TFA)
B: acetonitrile + 0.1% TFA

**Table A: HPLC gradient**

| Time (min) | A(%) | B(%) |
|---|---|---|
| 0 | 90 | 10 |
| 15 | 10 | 90 |
| 20 | 10 | 90 |
| 20.1 | 90 | 10 |
| 30 | 90 | 10 |

A particle size analysis is carried out on the Lecotrac laser particle size analyser and compared to the results from the starting material for comparison.

Formulations for pharmaceutical use incorporating compounds of the present invention either pre or post nanomilling can be prepared in various forms and with numerous excipients. Examples of such formulations are given

below.

**Example 116: Inhalant Formulation**

A compound of formula (I) or a pharmaceutically acceptable salt or solvate thereof, (1 mg to 100 mg) is aerosolized from a metered dose inhaler to deliver the desired amount of drug per use.

**Example 117: Tablet Formulation**

| Tablets/Ingredients | Per Tablet |
|---|---|
| 1. Active ingredient | 40 mg |
| (Compound of formula (I) or pharmaceutically acceptable salt or solvate) | |
| 2. Corn Starch | 20 mg |
| 3. Alginic acid | 20 mg |
| 4. Sodium Alginate | 20 mg |
| 5. Mg stearate | 1.3 mg |

Procedure for tablet formulation:

Ingredients 1, 2, 3 and 4 are blended in a suitable mixer/blender. Sufficient water is added portion-wise to the blend with careful mixing after each addition until the mass is of a consistency to permit its conversion to wet granules. The wet mass is converted to granules by passing it through an oscillating granulator using a No. 8 mesh (2.38 mm) screen. The wet granules are then dried in an oven at 140°F (60°C) until dry. The dry granules are lubricated with ingredient No. 5, and the lubricated granules are compressed on a suitable tablet press.

**Example 118: Parenteral Formulation**

A pharmaceutical composition for parenteral administration is prepared by dissolving an appropriate amount of a compound of formula (I) in polyethylene glycol with heating. This solution is then diluted with water for injections Ph Eur. (to 100 ml). The solution is then rendered sterile by filtration through a 0.22 micron membrane filter and sealed in sterile containers.

**Claims**

**1.** A compound selected from
2-(3-Chlorophenylamino)-4-trifluoromethylpyrimidine-5-carboxylic acid benzyl-amide;
2-(3-Chlorophenylamino)-4-trifluoxometllylpyrimidine-5-carboxylic acid (pyridin-4-ylmethyl)amide;
2-(3-Chlorophenylamino)-4-trifluoromethylpyrimidine-5-carboxylic acid N-benzyl-N-methylamide;
2-(3-Chlorophenylamino)-4-trifluoromethylpyrimidine-5-carboxylic acid 4-methoxybenzyl-amide;
2-(3-Chlorophenylamino)-4-trifluoromethylpyrimidine-5-carboxylic acid 4-fluorobenzyl-amide;
2-(3-Chlorophenylamino)-4-trifluoromethylpyrimidine-5-carboxylic acid 4-cyanobenzyl-amide;
2-(2,3-Dichlorophenylamino)-4-trifluoromethylpyrimidine-5-carboxylic acid N-benzyl-N-methylamide;
2-(2,4-Dichlorophenylamino)-4-trifluoromethylpyrimidine-5-carboxylic acid benzyl-amide;
2-(3,4-Dichlorophenylamino)-4-trifluoromethylpyrimidine-5-carboxylic acid benzyl-amide;
2-(2,6-Dichlorophenylamino)-4-trifluoromethylpyrimidine-5-carboxylic acid N-benzyl-N-methylamide;
2-(3,5-Dichlorophenylamino)-4-trifluoromethylpyrimidine-5-carboxylic acid benzyl-amide;
2-(3-Fluorophenylamino)-4-trifluoromethylpyrimidine-5-carboxylic acid benzyl-amide;
2-(3-Bromophenylamino)-4-trifluoromethylpyrimidine-5-carboxylic acid benzyl-amide;
2-(3-Bromophenylamino)-4-trifluoromethylpyrimidine-5-carboxylic acid N-benzyl-N-methylamide;
2-(2-Methoxyphenylamino)-4-trifluoromethylpyrimidine-5-carboxylic acid benzyl-amide;
2-(2,3-Dichlorophenylaznino)-4-trifluoromethylpyrimidine-5-carboxylic acid (pyridin-4-ylmethyl)amide;
2-(2,4-Dichlorophenylamino)-4-trifluoroznethylpyriznidine-5-carboxylic acid (pyridin-4-ylmethyl)amide;
2-(3,4-Dichlorophenylamino)-4-trifluoromethylpyrimidine,-5-carboxylic acid (pyridin-4-ylmethyl)amide;

2-(2,5-Dichlo.rophenylarn-mo)-4-trifluoromethylpyrimidine-5-carboxylic acid (pyridin-4-ylmethyl)amide;
2-(3-Fluorophenylarnino)-4-trifluoromothylpyriiDidine-5-carboxylic acid (pyridin-4-ylmethyl)amide;
2-(3-Bromophenylamino)-4-trifluoromethylpyrimidine-5-carboxylic acid (pyridin-4-ylmethyl)amide;
2-(3,5-Dichlorophenylamino)-4-trifluoromethylpyrimidine-5-carboxylic acid (pyridin-4-ylmethyl)amide;
2-(3-Fluorophenylamino)-4-trifluoromethylpyrimidine-5-carboxylic acid 4-fluorobenzyl-amide;
2-(3-Bromophenylamino)-4-trifluoromethylpyrimidine-5-carboxylic acid 4-fluorobenzyl-amide;
2-(3,5-Dichlorophenylamino)-4-trifluoromethylpyrimidine-5-carboxylic acid 4-fluorobenzylamide;
2-(3,5-Dichlorophenylamino)-4-trifluoromethylpyrimidine-5-carboxylic acid 4-cyanobenzyl-amide;
2-(4-Cyano-phenylamino)-4-trifluoromethyl-pyrimidine-5-carboxylic acid benzylamide;
2-(3-Chloro-phenylamino)-4-trifluoromethyl-pyrimidine-5-carboxylic acid (2-methylpyridin-4-ylmethyl)-amide hydrochloride; and
2-(3-Chloro-phenylamino)-4-trifluoromethyl-pyrimidine-5-carboxylic acid (2-fluoro-pyridin-4-ylmethyl)-amide;
or a pharmaceutically acceptable salt or solvate thereof.

**2.** A pharmaceutical composition comprising a compound as claimed in claim 1 or a pharmaceutically acceptable salt or solvate thereof.

**3.** Use of a compound as claimed in claim 1 or a pharmaceutically acceptable salt or solvate thereof in the manufacture of a medicament for treatment of a condition which is mediated by the activity of cannabinoid 2 receptors.

**4.** Use of claim 3 wherein the condition which is mediated by the activity of cannabinoid 2 receptors is pain, rheumatoid arthritis, or osteoarthritis.

## Patentansprüche

**1.** Verbindung, ausgewählt aus:

2-(3-Chlorphenylamino)-4-trifluormethylpyrimidin-5-carbonsäurebenzylamid;
2-(3-Chlorphenylamino)-4-trifluormethylpyrimidin-5-carbonsäure(pyridin-4-ylmethyl)amid;
2-(3-Chlorphenylamino)-4-trifluormethylpyrimidin-5-carbonsäure-N-benzyl-N-methylamid;
2-(3-Chlorphenylamino)-4-trifluormethylpyrimidin-5-carbonsäure-4-methoxybenzylamid;
2-(3-Chlorphenylamino)-4-trifluormethylpyrimidin-5-carbonsäure-4-fluorbenzylamid;
2-(3-Chlorphenylamino)-4-trifluormethylpyrimidin-5-carbonsäure-4-cyanobenzylamid;
2-(2,3-Dichlorphenylamino)-4-trifluormethylpyrimidin-5-carbonsäure-N-benzyl-N-methylamid;
2-(2,4-Dichlorphenylamino)-4-trifluormethylpyrimidin-5-carbonsäurebenzylamid;
2-(3,4-Dichlorphenylamino)-4-trifluormethylpyrimidin-5-carbonsäurebenzylamid;
2-(2,6-Dichlorphenylamino)-4-trifluormethylpyrimidin-5-carbonsäure-N-benzyl-N-methylamid;
2-(3,5-Dichlorphenylamino)-4-trifluormethylpyrimidin-5-carbonsäurebenzylamid;
2-(3-Fluorphenylamino)-4-trifluormethylpyrimidin-5-carbonsäurebenzylamid;
2-(3-Bromphenylamino)-4-trifluormethylpyrimidin-5-carbonsäurebenzylamid;
2-(3-Bromphenylamino)-4-trifluormethylpyrimidin-5-carbonsäure-N-benzyl-N-methylamid;
2-(2-Methoxyphenylamino)-4-trifluormethylpyrimidin-5-carbonsäurebenzylamid;
2-(2,3-Dichlorphenylamino)-4-trifluormethylpyrimidin-5-carbonsäure(pyridin-4-ylmethyl)amid;
2-(2,4-Dichlorphenylamino)-4-trifluormethylpyrimidin-5-carbonsäure(pyridin-4-ylmethyl)amid;
2-(3,4-Dichlorphenylamino)-4-trifluormethylpyrimidin-5-carbonsäure(pyridin-4-ylmethyl)amid;
2-(2,5-Dichlorphenylamino)-4-trifluormethylpyrimidin-5-carbonsäure(pyridin-4-ylmethyl)amid;
2-(3-Fluorphenylamino)-4-trifluormethylpyrimidin-5-carbonsäure(pyridin-4-ylmethyl)-amid;
2-(3-Bromphenylamino)-4-trifluormethylpyrimidin-5-carbonsäure(pyridin-4-ylmethyl)-amid;
2-(3,5-Dichlorphenylamino)-4-trifluormethylpyrimidin-5-carbonsäure(pyridin-4-ylmethyl)amid;
2-(3-Fluorphenylamino)-4-trifluormethylpyrimidin-5-carbonsäure-4-fluorbenzylamid;
2-(3-Bromphenylamino)-4-trifluormethylpyrimidin-5-carbonsäure-4-fluorbenzylamid;
2-(3,5-Dichlorphenylamino)-4-trifluomethylpyrimidin-5-carbonsäure-4-fluorbenzy]-amid;
2-(3,5-Dichlorphenylamino)-4-trifluormethylpyrimidin-5-carbonsäure-4-cyanobenzylamid;
2-(4-Cyanophenylamino)-4-trifluormethylpyrimidin-5-carbonsäurebenzylamid;
2-(3-Chlorphenylamino)-4-trifluormethylpyrimidin-5-carbonsäure-(2-methylpyridin-4-ylmethyl)amidhydrochlorid; und
2-(3-Chlorphenylamino)-4-trifluormethylpyrimidin-5-carbonsäure-(2-fluorpyridin-4-ylmethyl)amid;

oder ein pharmazeutisch verträgliches Salz oder Solvat davon.

2. Arzneimittel, umfassend eine Verbindung gemäß Anspruch 1 oder ein pharmazeutisch verträgliches Salz oder Solvat davon.

3. Verwendung einer Verbindung gemäß Anspruch 1 oder eines pharmazeutisch verträglichen Salzes oder Solvats davon zur Herstellung eines Medikaments zur Behandlung eines durch die Cannabinoid-2-Rezeptorenaktivität vermittelten Zustands.

4. Verwendung gemäß Anspruch 3, wobei der Zustand, welcher durch die Cannabinoid-2-Rezeptorenaktivität vermittelt wird, Schmerz., theumatoide Arthritis oder Osteoarthritis ist.

**Revendications**

1. Composé choisi entre
le benzyl-amide d'acide 2-(3-chlorophénylamino)-4-trifluorométhylpyrimidine-5-carboxylique ;
le (pyridine-4-ylméthyl)amide d'acide 2-(3-chlorophénylamino) -4-trifluorométhylpyrimidine-5-carboxylique ;
le N-benzyl-N-méthylamide d'acide 2-(3-chlorophénylamino)-4-trifluorométhylpyrimidine-5-carboxylique ;
le 4-méthoxybenzyl-amide d'acide 2-(3-chlorophénylamino)-4-trifluorométhylpyrimidine-5-carboxylique ;
le 4-fluorobenzyl-amide d'acide 2-(3-chlorophénylamino)-4-trifluorométhylpyrimidine-5-carboxylique ;
le 4-cyanobenzyl-amide d'acide 2-(3-chlorophénylamino)-4-trifluorométhylpyrimidine-5-carboxylique ;
le N-benzyl-N-méthylamide d'acide 2-(2,3-dichlorophénylamino)-4-trifluorométhylpyrimidine-5-carboxylique ;
le benzyl-amide d'acide 2-(2,4-dichlorophénylamino)-4-trifluorométhylpyrimidine-5-carboxylique ;
le benzyl-amide d'acide 2-(3,4-dichlorophénylamino)-4-trifluorométhylpyrimidine-5-carboxylique ;
le N-benzyl-N-méthylamide d'acide 2-(2,6-dichlorophénylamino)-4-trifluorométhylpyrimidine-5-carboxylique ;
le benzyl-amide d'acide 2-(3,5-dichlorophénylamino)-4-trifluorométhylpyrimidine-5-carboxylique ;
le benzyl-amide d'acide 2-(3-fluorophénylamino)-4-trifluorométhylpyrimidine-5-carboxylique ;
le benzyl-amide d'acide 2-(3-bromophénylamino)-4-trifluorométhylpyrimidine-5-carboxylique ;
le N-benzyl-N-méthylamide d'acide 2-(3-bromophénylamino)-4-trifluorométhylpyrimidine-5-carboxylique ;
le benzyl-amide d'acide 2-(2-méthoxyphénylamino)-4-trifluorométhylpyrimidine-5-carboxylique ;
le (pyridine-4-ylméthyl)amide d'acide 2-(2,3-dichlorophénylamino)-4-trifluorométhylpyrimidine-5-carboxylique ;
le (pyridine-4-ylméthyl)amide d'acide 2-(2,4-dichlorophénylamino) -4-trifluorométhylpyrimidine-5-carboxylique ;
le (pyridine-4-ylméthyl)amide d'acide 2-(3,4-dichlorophénylamino)-4-trifluorométhylpyrimidine-5-carboxylique ;
le (pyridine-4-ylméthyl)amide d'acide 2-(2,5-dichlorophénylamino)-4-trifluorométhylpyrimidine-5-carboxylique ;
le (pyridine-4-ylméthyl)amide d'acide 2-(3-f luorophénylamino)-4-trifluorométhylpyrimidine-5-carboxylique ;
le (pyridine-4-ylméthyl)amide d'acide 2-(3-bromophénylamino)-4-trifluorométhylpyrimidine-5-carboxylique ;
le (pyridine-4-ylméthyl)amide d'acide 2-(3,5-dichlorophénylamino) -4-trifluorométhylpyrimidine-5-carboxylique ;
le 4-fluorobenzyl-amide d'acide 2-(3-fluorophénylamino)-4-trifluorométhylpyrimidine-5-carboxylique ;
le 4-fluorobenzyl-amide d'acide 2-(3-bromophénylamino)-4-trifluorométhylpyrimidine-5-carboxylique ;
le 4-fluorobenzylamide d'acide 2-(3,5-dichlorophénylamino)-4-trifluorométhylpyrimidine-5-carboxylique ;
le 4-cyanobenzyl-amide d'acide 2-(3,5-dichlorophénylamino)-4-trifluorométhylpyrimidine-5-carboxylique;
le benzylamide d'acide 2-(4-cyano-phénylamino)-4-trifluorométhyl-pyrimidine-5-carboxylique ;
le chlorhydrate de (2-méthyl-pyridine-4-ylméthyl)-amide d'acide 2-(3-chloro-phénylamino)-4-trifluorométhyl-pyrimidine-5-carboxylique ; et
le (2-fluoro-pyridine-4-ylméthyl)-amide d'acide 2-(3-chloro-phénylamino)-4-trifluorométhyl-pyrimidine-5-carboxylique ;
ou un de ses sels ou produits de solvatation pharmaceutiquement acceptables.

2. Composition pharmaceutique comprenant un composé suivant la revendication 1 ou un de ses sels ou produits de solvatation pharmaceutiquement acceptables.

3. Utilisation d'un composé suivant la revendication 1 ou d'un de ses sels ou produits de solvatation pharmaceutiquement acceptables dans la production d'un médicament destiné au traitement d'une affection qui est soumise à une médiation par l'activité des récepteurs de cannabinoïdes 2.

4. Utilisation suivant la revendication 3, dans laquelle l'affection qui est soumise à une médiation par l'activité de récepteurs de cannabinoïdes 2 est la douleur, la polyarthrite rhumatoïde ou l'arthrose.